(19) Europäisches Patentamt
European Patent Office
Office européen des brevets

(11) Veröffentlichungsnummer: 0 070 798 A2

(12) **EUROPÄISCHE PATENTANMELDUNG**

(21) Anmeldenummer: **82810277.2**

(22) Anmeldetag: **28.06.82**

(51) Int. Cl.³: **C 07 D 249/08**, C 07 D 233/60, A 01 N 43/64, A 01 N 43/50 // C07C49/175, C07D303/22, C07D303/34, C07C49/84

(30) Priorität: **02.07.81 CH 4375/81**
**05.04.82 CH 2079/82**

(43) Veröffentlichungstag der Anmeldung: **26.01.83 Patentblatt 83/4**

(84) Benannte Vertragsstaaten: **AT BE CH DE FR IT LI LU NL SE**

(71) Anmelder: **CIBA-GEIGY AG, Patentabteilung Postfach, CH-4002 Basel (CH)**

(72) Erfinder: **Meyer, Alfred, Dr., Bristenweg 6, CH-4054 Basel (CH)**
Erfinder: **Nyfeler, Robert, Dr., Bärenfelserstrasse 8, CH-4057 Basel (CH)**
Erfinder: **Sturm, Elmar, Dr., Klusstrasse 66, CH-4147 Aesch (CH)**
Erfinder: **Müller, Urs, Dr., Schluchtstrasse 15, CH-4142 Münchenstein (CH)**

(54) **Mikrobizide und wuchsregulierende Mittel.**

(57) Es werden neue 1-Azolyl-2-hydroxy-alkan-derivate der allgemeinen Formel I

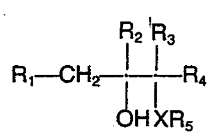

beschrieben, worin

$R_1$ für eine Azolylgruppe steht;

$R_2$ $C_1$-$C_4$-Alkyl, ein unsubstituiertes oder durch Halogen, Cyano, $C_1$-$C_3$-Alkyl, $C_1$-$C_3$-Alkoxy, Nitro, Rhodano, $C_1$-$C_3$-Alkylthio und/oder $C_1$-$C_3$-Haloalkyl substituiertes Aryl bedeutet:

$R_3$ und $R_4$ unabhängig voneinander für $C_1$-$C_4$-Alkyl stehen oder einer von beiden für Wasserstoff und der andere für $C_1$-$C_4$-Alkyl steht:

$R_5$ für einen unsubstituierten oder ein- oder mehrfach substituierten Rest steht, ausgewählt aus der Reihe Phenyl, Biphenyl, Benzylphenyl, Benzyloxyphenyl und Phenoxyphenyl, wobei die Substituenten aus der Reihe Halogen, Cyano, $C_1$-$C_3$-Alkyl, $C_1$-$C_5$-alkoxy, $C_1$-$C_5$-Haloalkyl, $C_1$-$C_3$-Alkylthio, $C_1$-$C_3$-Haloalkyl, $C_1$-$C_3$-Haloalkylthio, Nitro und/oder Rhodano ausgewählt sind; und

X Sauerstoff oder Schwefel bedeutet; unter Einschluss der Säureadditionssalze, quaternären Azoliumsalze und Metallkomplexe.

Es werden ferner Methoden zur Herstellung dieser Produkte offenbart sowie agrochemische Mittel, die als Wirkstoff eine dieser Verbindungen enthalten. Ferner wird ein Verfahren zur Bekämpfung phytopathogener Mikroorganismen und/oder zur Regulierung des Pflanzenwuchses mit Hilfe dieser Substanzen beschrieben.



ACTORUM AG

CIBA-GEIGY AG

Basel (Schweiz)

Case 5-13469/1+2/B/+

## Mikrobizide und wuchsregulierende Mittel

Die vorliegende Erfindung betrifft neue, substituierte 1-Azolyl-2-hydroxy-alkan-derivate sowie deren Säureadditionssalze, quaternäre Azoliumsalze und Metallkomplexe. Die Erfindung betrifft ferner die Herstellung dieser Substanzen sowie mikrobizide und wuchsregulierende Mittel, die als Wirkstoff mindestens eine dieser Verbindungen enthalten. Die Erfindung betrifft auch die Herstellung der genannten Mittel und die Verwendung der Wirkstoffe oder der Mittel zur Regulierung des Pflanzenwachstums und zur Bekämpfung von schädlichen Mikroorganismen.

Es werden hierin Verbindungen der allgemeinen Formel I

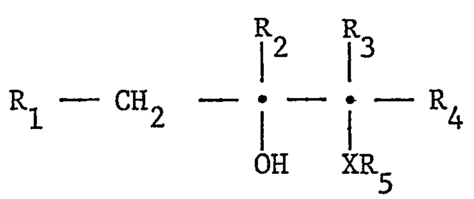

(I)

umfasst, worin

$R_1$ für eine Azolylgruppe steht;

$R_2$ $C_1$-$C_4$-Alkyl, ein unsubstituiertes oder durch Halogen, Cyano, $C_1$-$C_3$-Alkyl, $C_1$-$C_3$-Alkoxy, Nitro, Rhodano, $C_1$-$C_3$-Alkylthio und/oder $C_1$-$C_3$-Haloalkyl substituiertes Aryl bedeutet;

$R_3$ und $R_4$ unabhängig voneinander für $C_1$-$C_4$-Alkyl stehen oder einer von beiden für Wasserstoff und der andere für $C_1$-$C_4$-Alkyl steht;

$R_5$ für einen unsubstituierten oder ein- oder mehrfach substituierten Rest steht, ausgewählt aus der Reihe Phenyl, Biphenyl, Benzylphenyl, Benzyloxyphenyl und Phenoxyphenyl, wobei die Substituenten aus der Reihe Halogen, Cyano, $C_1$-$C_3$-Alkyl, $C_1$-$C_5$-Alkoxy, $C_1$-$C_5$-Haloalkoxy,

$C_1$-$C_3$-Alkylthio, $C_1$-$C_3$-Haloalkyl, $C_1$-$C_3$-Haloalkylthio, Nitro und/ oder Rhodano ausgewählt sind; und

X Sauerstoff oder Schwefel bedeutet; unter Einschluss der Säureadditionssalze, quaternären Azoliumsalze und Metallkomplexe.

Der Ausdruck Azolyl kennzeichnet einen fünfgliedrigen heterocyclischen Fünfring mit Stickstoff als Heteroatom und mit aromatischem Charakter. Typische Vertreter sind 1H-1,2,4-Triazol, 4H-1,2,4-Triazol und 1H-Imidazol. Unter dem Begriff Alkyl selbst oder als Bestandteil eines anderen Substituenten sind je nach Zahl der angegebenen Kohlenstoffatome beispielsweise folgende Gruppen zu verstehen: Methyl, Ethyl, Propyl, Butyl, Pentyl, Hexyl, Heptyl, Octyl usw. sowie ihre Isomeren, wie z.B. Isopropyl, Isobutyl, tert.-Butyl, Isopentyl usw.. Haloalkyl steht für einen einfach bis perhalogenierten Alkylsubstituenten, wie z.B. $CHCl_2$, $CHF_2$, $CH_2Cl$, $CCl_3$, $CH_2F$, $CH_2CH_2Cl$, $CHBr_2$ usw. Unter Halogen soll hier und im folgenden Fluor, Chlor, Brom oder Jod, vorzugsweise Fluor, Chlor oder Brom, verstanden werden. Naphthyl steht für α- oder β-Naphthyl, vorzugsweise α-Naphthyl. Der Ausdruck Alkylen steht für eine unverzweigte oder verzweigte Alkylenbrücke wie z.B. Methylen, Ethylen, Propylen usw., bevorzugt für ein Brückenglied mit 4 bis 6 Kohlenstoffatomen. Aryl bedeutet z.B. Naphtyl, insbesondere Phenyl und Aralkyl einen Niederalkylrest, der durch eine aromatische Gruppe substituiert ist. Cycloalkyl bedeutet je nach Zahl der Kohlenstoffatome z.B. Cyclopropyl, Cyclobutyl, Cyclopentyl, Cyclohexyl, Cycloheptyl, Cyclooctyl usw.

Die vorliegende Erfindung betrifft somit die freien organischen Moleküle der Formel I, deren Säureadditionssalze, quaternären Azoliumsalze und Metallkomplexe. Die freien Moleküle sind bevorzugt.
Beispiele salzbildender Säuren sind anorganische Säuren: Halogenwasserstoffsäure wie Fluorwasserstoffsäure, Chlorwasserstoffsäure, Bromwasserstoffsäure oder Jodwasserstoffsäure sowie Schwefelsäure, Phosphorsäure, phosphorige Säure, Salpetersäure und organische Säuren wie Essigsäure, Trifluoressigsäure, Trichloressigsäure, Propionsäure, Glycolsäure, Thiocyansäure, Milchsäure, Bernsteinsäure, Zitronensäure, Benzoesäure, Zimtsäure, Oxalsäure, Ameisensäure,

Benzolsulfonsäure, p-Toluolsulfonsäure, Methansulfonsäure, Salicylsäure, p-Aminosalicylsäure, 2-Phenoxybenzoesäure oder 2-Acetoxybenzoesäure.

Metallkomplexe der Formel I bestehen aus dem zugrundeliegenden organischen Molekül und einem anorganischen oder organischen Metallsalz, beispielsweise den Halogeniden, Nitraten, Sulfaten, Phosphaten, Acetaten, Trifluoracetaten, Trichloracetaten, Propionaten, Tartraten, Sulfonaten, Salicylaten, Benzoaten usw. der Elemente der dritten und vierten Hauptgruppe wie Aluminium, Zinn oder Blei sowie der ersten bis achten Nebengruppe wie Chrom, Mangan, Eisen, Kobalt, Nickel, Kupfer, Zink, Silber, Quecksilber usw. Bevorzugt sind die Nebengruppen-Elemente der 4. Periode. Die Metalle können dabei in den verschiedenen ihnen zukommenden Wertigkeiten vorliegen. Die Metallkomplexe der Formel I können ein- oder mehrkernig auftreten, d.h. sie können ein oder mehrere organische Molekülanteile als Liganden enthalten. Komplexe mit den Metallen Kupfer, Zink, Mangan und Zinn sind bevorzugt.

Die Verbindungen der Formel I sind bei Raumtemperatur stabile Oele, Harze oder überwiegend Feststoffe, die sich durch sehr wertvolle mikrobizide und wuchsregulierende Eigenschaften auszeichnen. Sie lassen sich auf dem Agrarsektor oder verwandten Gebieten präventiv und kurativ zur Bekämpfung von pflanzenschädigenden Mikroorganismen und zur Regulierung des Pflanzenwuchses einsetzen, dabei sind die Triazolylmethylderivate im Umfang der Formel I bevorzugt. Die erfindungsgemässen Wirkstoffe der Formel I zeichnen sich durch eine sehr gute Verträglichkeit bei Kulturpflanzen aus.

Aufgrund ihrer ausgeprägten wuchsregulierenden und/oder mikrobiziden Wirkung sind diejenigen Wirksubstanzen der Formel I bevorzugt, die folgende Substituententypen oder Kombinationen dieser Substituententypen untereinander aufweisen:

Bei $R_1$: a) 1H-1,2,4-Triazol, 4H -1,2,4-Triazol, 1H-Imidazol.

b) 1H-1,2,4-Triazol, 4H-1,2,4-Triazol.

c) 1H-1,2,4-Triazol.

Bei $R_2$: a) Methyl, Ethyl, iso-Propyl, tert.-Butyl, Phenyl, durch $C_1$-$C_3$-Alkyl, $C_1$-$C_3$-Alkoxy, $CF_3$, Halogen und/oder Phenyl substituiertes Phenyl.

b) Methyl, iso-Propyl, tert.-Butyl, Phenyl, Halophenyl, Dih alophenyl, Biphenyl.

c) tert.-Butyl, Halophenyl, Dihalophenyl.

d) tert.-Butyl, 4-Chlorphenyl, 4-Bromphenyl, 2,4-Dichlorphenyl, 2-Chlor-4-Bromphenyl.

e) tert.-Butyl, 4-Chlorphenyl, 2,4-Dichlorphenyl.

f) tert.-Butyl, 2,4-Dichlorphenyl.

g) tert.-Butyl.

Bei $R_3$ und $R_4$

a) unabhängig voneinander $C_1$-$C_2$-Alkyl oder einer von beiden H und der andere $C_1$-$C_4$-Alkyl.

b) unabhängig voneinander $C_1$-$C_4$-Alkyl.

c) $R_3$ = H, $CH_3$, $C_2H_5$; $R_4$ = $CH_3$, $C_2H_5$.

d) $R_3$ = H, $R_4$ = $C_1$-$C_2$-Alkyl.

Bei X: a) Sauerstoff, Schwefel.

b) Sauerstoff.

Bei $R_5$: a) ein unsubstituierter oder ein- oder mehrfach durch Halogen, Cyano, $C_1$-$C_2$-Alkyl, $C_1$-$C_2$-Alkoxy, $C_1$-$C_5$-Haloalkoxy, $C_1$-$C_2$-Alkylthio, $C_1$-$C_2$-Halogenalkyl, $C_1$-$C_2$-Haloalkylthio und/oder Nitro substituierter Rest aus der Reihe Phenyl, Biphenyl, Benzylphenyl und Phenoxyphenyl.

b) ein unsubstituierter oder ein- oder mehrfach durch $C_1$-$C_2$-Alkyl, $C_1$-$C_2$-Alkoxy, Halogen, Cyano, $CF_3$, Nitro, und/oder $C_1$-$C_2$-Alkylthio substituierter aromatischer Rest aus der Reihe Phenyl, Biphenyl, Benzylphenyl, Benzoxyphenyl und Phenoxyphenyl.

c) ein unsubstituiertes oder ein- bis dreifach durch $C_1$-$C_2$-Alkyl, $C_1$-$C_2$-Alkoxy, Halogen, Cyano, $CF_3$, Nitro und/oder $C_1$-$C_2$-Alkylthio substituiertes Phenyl.

d) ein unsubstituiertes oder ein- bis dreifach durch Methyl, Methoxy, Chlor, Brom, Fluor, Cyano, $CF_3$, Nitro oder Methylthio substituiertes Phenyl.

Es ergeben sich somit z.B. folgende bevorzugte Gruppen:

Eine bevorzugte Gruppe bilden Verbindungen der Formel I, worin $R_1$ für eine 1H-1,2,4-Triazolyl-, 4H-1,2,4-Triazolyl- oder eine 1H-Imidazolylgruppe steht; $R_2$ Methyl, Ethyl, iso-Propyl, tert.-Butyl, unsubstituiertes oder durch $C_1$-$C_3$-Alkyl, $C_1$-$C_3$-Alkoxy, $CF_3$, Halogen und/oder Phenyl ein- oder mehrfach substituiertes Phenyl bedeutet; $R_3$ und $R_4$ unabhängig voneinander für $C_1$-$C_2$-Alkyl stehen oder einer von beiden für Wasserstoff und der andere für $C_1$-$C_4$-Alkyl stehen; $R_5$ für einen unsubstituierten oder ein- oder mehrfach durch Halogen, Cyano, $C_1$-$C_2$-Alkyl, $C_1$-$C_2$-Alkoxy, $C_1$-$C_5$-Haloalkoxy, $C_1$-$C_2$-Alkylthio, $C_1$-$C_2$-Haloalkyl, $C_1$-$C_2$-Halothioalkyl und/oder Nitro substituierten Rest darstellt, ausgewählt aus der Reihe Phenyl, Biphenyl, Benzylphenyl und Phenoxyphenyl.

Bevorzugt werden Verbindungen der Formel I, worin $R_1$ für eine 1H-1,2,4-Triazolyl-, 4H-1,2,4-Triazolyl- oder eine 1H-Imidazolylgruppe steht; $R_2$ Methyl, Ethyl, iso-Propyl, tert.-Butyl, unsubstituiertes oder durch $C_1$-$C_3$-Alkyl, $C_1$-$C_3$-Alkoxy, $CF_3$, Halogen und/oder Phenyl ein- oder mehrfach substituiertes Phenyl bedeutet; $R_3$ und $R_4$ unabhängig voneinander für $C_1$-$C_4$-Alkyl stehen; $R_5$ für einen unsubstituierten oder ein- oder mehrfach durch $C_1$-$C_2$-Alkyl, $C_1$-$C_2$-Alkoxy, Halogen, Cyano, $CF_3$, Nitro und/oder $C_1$-$C_2$-Alkylthio substituierten aromatischen Rest, ausgewählt aus der Reihe Phenyl, Biphenyl, Benzylphenyl, Benzoxyphenyl und Phenoxyphenyl steht und X Sauerstoff oder Schwefel bedeutet, unter Einschluss der pflanzenverträglichen Säureadditionssalze, quaternären Azoliumsalzen und Metallkomplexe.

Eine weitere bevorzugte Gruppe bilden Verbindungen der Formel I, worin $R_1$ für eine 1H-1,2,4-Triazolyl-, 4H-1,2,4-Triazolyl- oder eine 1H-Imidazolylgruppe steht; $R_2$ Methyl, iso-Propyl, tert.-Butyl, Phenyl, Halophenyl, Dihalophenyl oder Biphenyl bedeutet; $R_3$ Waserstoff bedeutet und $R_4$ für $C_1$-$C_3$-Alkyl steht; X Sauerstoff oder Schwefel bedeuten; und $R_5$ einen unsubstituierten oder ein- oder mehrfach durch $C_1$-$C_2$-Alkyl, $C_1$-$C_2$-Alkoxy, Halogen, Cyano, $CF_3$, Nitro und/oder $C_1$-$C_2$-Alkylthio substituierten aromatischen Rest, ausgewählt aus der Reihe Phenyl, Biphenyl, Benzylphenyl, Benzoxyphenyl und Phenoxyphenyl steht.

Eine besonders interessante Gruppe bilden Verbindungen der Formel I, worin $R_1$ für eine 1H-1,2,4-Triazolyl- oder eine 4H-1,2,4-Triazolylgruppe steht; $R_2$ tert.-Butyl, Halophenyl oder Dihalophenyl bedeutet; $R_3$ Wasserstoff, Methyl oder Ethyl bedeutet; $R_4$ Methyl oder Ethyl bedeutet; $R_5$ für ein unsubstituiertes oder ein- bis dreifach durch $C_1$-$C_2$-Alkyl, $C_1$-$C_2$-Alkoxy, Halogen, Cyano, $CF_3$, Nitro und/oder $C_1$-$C_2$-Alkylthio substituiertes Phenyl steht und X Sauerstoff oder Schwefel bedeutet.

Innerhalb der letztgenannten Gruppe werden insbesondere jene Verbindungen der Formel I bevorzugt, worin $R_1$ für 1H-1,2,4-Triazol oder 4H-1,2,4-Triazol steht; $R_2$ tert.-Butyl, 4-Chlorphenyl, 4-Bromphenyl, 2,4-Dichlorphenyl oder 2-Chlor-4-Bromphenyl bedeutet; $R_3$ Wasserstoff und $R_4$ Methyl oder Ethyl bedeuten; $R_5$ für ein unsubstituiertes oder ein- bis dreifach durch Methyl, Methoxy, Chlor, Brom, Fluor, Cyano, $CF_3$, Nitro oder Methylthio substituiertes Phenyl steht und X Sauerstoff bedeutet.

Unter den wuchsregulierenden Substanzen sind insbesondere jene im Umfang der Formel I bevorzugt, worin $R_2$ für einen $C_1$-$C_4$-Alkylrest, insbesondere den tert.-Butylrest steht.

Unter den mikrobizid wirkenden Substanzen sind jene Verbindungen der Formel I besonders bevorzugt, worin $R_2$ für gegebenenfalls substituiertes Aryl, insbesondere mono- oder dihalogeniertes Phenyl steht, wobei als Halogen vor allem Chlor und/oder Fluor vorteilhaft ist.

Verbindungen im Unfang der Formel I, worin $R_3$ und $R_4$ für Methyl stehen, bilden auch eine bevorzugte Wirkstoffgruppe.

Folgende Einzelsubstanzen werden besonders bevorzugt:

1-[1H-1,2,4-Triazolyl]-2-methyl-2-hydroxy-3-[4-chlorphenoxy]-3-methyl-butan,

1-[1H-1,2,4-Triazolyl]-2-methyl-2-hydroxy-3-[2,4-dichlorphenoxy]-3-methyl-butan,

1-[1H-1,2,4-Triazolyl]-2-methyl-2-hydroxy-3-[3,4-dichlorphenoxy]-3-methyl-butan,

1-[1H-1,2,4-Triazolyl]-2-methyl-2-hydroxy-3-[4-biphenyl]-3-methyl-butan,

1-[1H-1,2,4-Triazolyl]-2-methyl-2-hydroxy-3-[4-isopropylphenoxy]-3-methyl-butan,

1-[1H-1,2,4-Triazolyl]-2-[2,4-dichlorphenyl]-2-hydroxy-3-[4-chlor-phenoxy]-pentan,

1-[1H-1,2,4-Triazolyl]-2-[2,4-dichlorphenyl]-2-hydroxy-3-[4-fluorpheno-xy]-pentan,

1-[1H-1,2,4-Triazolyl]-2-[2,4-dichlorphenyl]-2-hydroxy-3-[2,3-dimethyl-phenoxy]-pentan,

1-[1H-1,2,4-Triazolyl]-2-[2,4-dichlorphenyl]-2-hydroxy-3-[4-chlorphen-oxy]-hexan,

1-[1H-1,2,4-Triazolyl]-2-[2,4-dichlorphenyl]-2-hydroxy-3-[phenoxy]-pentan,

1-[1H-1,2,4-Triazolyl]-2-[2,4-dichlorphenyl]-2-hydroxy-3-[4-bromphenoxy]-pentan,

1-[1H-1,2,4-Triazolyl]-2-[2-chlor-4-fluorphenyl]-2-hydroxy-3-[4-chlorphenoxy]-butan,

1-[1H-1,2,4-Triazolyl]-2-tert.-butyl-2-hydroxy-3-[4-chlorphenoxy]-butan,

1-[1H-1,2,4-Triazolyl]-2-tert.-butyl-2-hydroxy-3-[4-fluorphenoxy]-butan,

1-[1H-1,2,4-Triazolyl]-2-tert.-butyl-2-hydroxy-3-[4-methylphenoxy]-buran,

1-[1H-1,2,4-Triazolyl]-2-tert.-butyl-2-hydroxy-3-[4-chlorphenoxy]-pentan,

1-[4H-1,2,4-Triazolyl]-2-tert.-butyl-2-hydroxy-3-[4-chlorphenoxy]-butan,

1-[4H-1,2,4-Triazolyl]-2-tert.-butyl-2-hydroxy-3-[4-fluorphenoxy]-butan,

1-[4H-1,2,4-Triazolyl]-2-[2,4-dichlorphenyl]-2-hydroxy-3-[4-chlorphenoxy]-butan.

Die Verbindungen der Formel I werden durch Reaktion eines Oxirans II

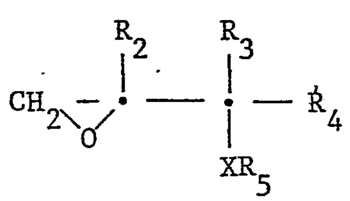

(II),

worin $R_2$, $R_3$, $R_4$, X und $R_5$ die unter Formel I angegebenen Bedeutungen haben, mit einem Azol der Formel III hergestellt

$$M - R_1 \qquad (III),$$

worin M für Wasserstoff oder bevorzugt ein Metallatom, insbesondere ein Alkalimetallatom, bedeutet und $R_1$ die unter Formel I angegebenen Bedeutungen hat.

Die Reaktion wird vorteilhafterweise in Gegenwart von Kondensationsmitteln oder von säurebindenden Mitteln durchgeführt. Als solche kommen organische und anorganische Basen in Betracht, z.B. tertiäre Amine wie Trialkylamine (Trimethylamin, Triethylamin, Tripropylamin usw.), Pyridin und Pyridinbasen (4-Dimethylaminopyridin, 4-Pyrrolidylaminopyridin usw.), Oxide, Hydride und Hydroxide, Carbonate und Hydrogencarbonate von Alkali- und Erdalkalimetallen (CaO, BaO, NaOH, LiOH, KOH, NaH, $Ca(OH)_2$, $KHCO_3$, $NaHCO_3$, $Ca(HCO_3)_2$, $K_2CO_3$,$Na_2CO_3$), sowie Alkaliacetate wie $CH_3COONa$ oder $CH_3COOK$. Darüberhinaus eignen sich auch Alkalialkoholate wie $C_2H_5ONa$, $C_3H_7$-nONa usw. In einigen Fällen kann es von Vorteil sein, wenn man das freie Azol III (M = Wasserstoff) zuerst, z.B. in situ mit einem Alkoholat, in das entsprechende Salz überführt und anschliessend in Gegenwart einer der genannten Basen mit dem Oxiran der Formel II umsetzt. Bei der Herstellung der 1,2,4-Triazolylderivate entstehen im allgemeinen parallel auch 1,3,4-Triazolylisomere, die sich auf übliche Weise, z.B. mit unterschiedlichen Lösungsmitteln, voneinander trennen lassen. Entstehender Halogenwasserstoff kann in manchen Fällen auch mittels Durchleiten von Inertgas, z.B. Stickstoff, aus dem Reaktionsgemisch vertrieben oder durch Zugabe von Molekularsieb absorbiert werden.

Die Reaktion (II mit III) wird bevorzugt in einem relativ polaren, jedoch reaktionsinerten organischen Lösungsmittel, durchgeführt, z.B. N,N-Dimethylformamid, N,N-Dimethylacetamid, Dimethylsulfoxid, Acetonitril, Benzonitril und anderen. Derartige Lösungsmittel können in Kombination mit anderen reaktionsinerten Lösungsmitteln, z.B. Benzol, Toluol, Xylol, Hexan, Petrolether, Chlorbenzol, Nitrobenzol u.a. durchgeführt werden. Die Reaktionstemperaturen liegen in einem Temperaturbereich von 0° bis 150°C, vorzugsweise 20° bis 100°C.

Im übrigen kann diese Reaktion analog zu bereits bekannten Umsetzungen von anderen Oxiranen mit Azolen [vgl. EP-OS 0 015 756 und DE-OS 29 12 288] durchgeführt werden.

Die Azole der Formel III sind bekannt oder werden nach an sich bekannten Methoden hergestellt.

Die Oxirane der Formel II sind neu, sie stellen besonders entwickelte Zwischenprodukte zur Herstellung der wertvollen Wirkstoffe der Formel I dar. Aufgrund ihrer strukturellen Beschaffenheit lassen sie sich in einfacher Weise in die Verbindungen der Formel I überführen.

Die Oxirane der Formel II lassen sich durch Reaktion der zugrundeliegenden Ketone der Formel IV

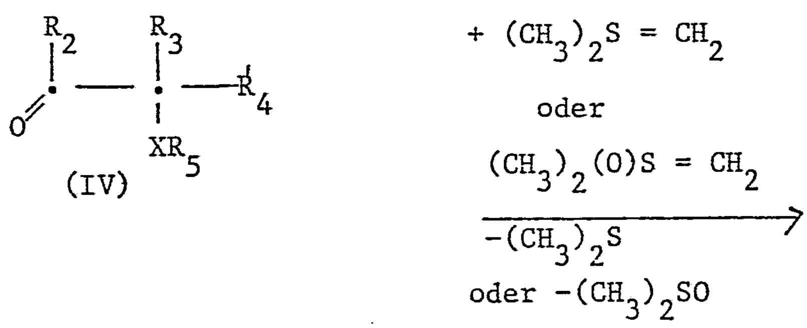


(IV) (II)

mit Dimethylsulfoniummethylid oder Dimethyloxosulfoniummethylid in Dimethylsulfoxid oder einem anderen geeigneten Lösungsmittel herstellen. In Formel IV haben die Substituenten die unter Formel I angegebenen Bedeutungen. Die Reaktion wird bei Temperaturen von 0° bis 120°C durchgeführt.

Analoge Reaktionen sind aus der Literatur bekannt; vgl. JACS, 87,1353 (1965) und Angew. Chem., 85, 867 (1973). Man kann die Reaktion prinzipiell analog zu den dort beschriebenen Umsetzungen vornehmen.

Ketone der Formel IV können aus den an sich bekannten α-Halogenketonen der Formel V

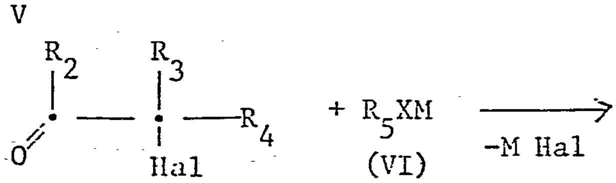


(V) (VI) (IV)

mit Verbindungen der Formel VI in üblichen reaktionsinerten Lösungsmitteln und gegebenenfalls bei erhöhter Temperatur hergestellt werden. In den Formeln V und VI haben die Substituenten $R_2$, $R_3$, $R_4$, $R_5$ und X die unter Formel I angegebenen Bedeutungen, Hal steht für Halogen, vorzugsweise für Chlor oder Brom und M bedeutet vorzugsweise ein Metallatom, insbesondere Natrium oder Kalium.

Ketone der Formel IV' lassen sich gemäss folgendem Schema auch

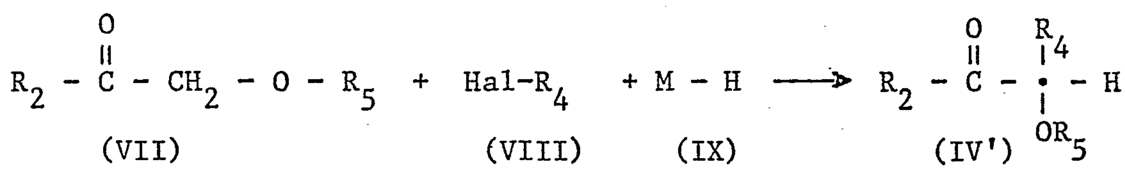

(VII) (VIII) (IX) (IV')

aus Ketonen der Formel VII durch Umsetzung mit Halogeniden der Formel VIII und einem Alkalihydrid der Formel IX herstellen, dabei stehen $R_2$, $R_4$ und $R_5$ für die unter Formel I aufgeführten Substituenten, Hal steht für ein Halogen, bevorzugt Chlor oder Brom und M bedeutet ein Alkalimetallatom, vorzugsweise Kalium oder Natrium. Die Reaktion wird im allgemeinen in einem üblichen reaktionsinerten Lösungsmittel bei Temperaturen von ca. 30-100°C durchgeführt.

Die Verbindungen der Formeln V bis IX sind bekannt, zum Teil käuflich oder können nach an sich bekannten Methoden hergestellt werden.

Bei der Herstellung aller hierin genannten Ausgangs-, Zwischen- und Endprodukte können grundsätzlich, sofern nicht ausdrücklich im einzelnen spezifiziert, ein oder mehrere reaktionsinerte Lösungs- oder Verdünnungsmittel anwesend sein. In Frage kommen beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander. In manchen Fällen kann es auch

von Vorteil sein, wenn die Reaktion oder Teilschritte einer Reaktion unter Schutzgasatmosphäre und/oder absoluten Lösungsmitteln durchgeführt werden. Als Schutzgase eignen sich inerte Gase wie Stickstoff, Helium, Argon oder in gewissen Fällen auch Kohlendioxid.

Das beschriebene Herstellungsverfahren ist, einschliesslich aller Teilschnitte, ein wichtiger Bestandteil der vorliegenden Erfindung.

Die Verbindungen der Formel I

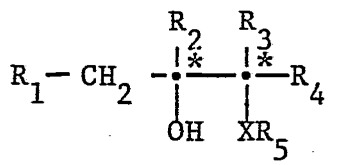

(I)

besitzen in Nachbarstellung zur Hydroxygruppe ein asymmetrisches Kohlenstoffatom (*) und bei unterschiedlichen Substituenten $R_3$ und $R_4$ ein weiteres benachbart zur $XR_5$-Gruppe. Entsprechend fallen die Produkte der Formel I üblicherweise als Enantiomeren- und Diastereomerengemische an und können entweder anschliessend nach üblichen Methoden in die reinen Isomeren aufgetrennt oder durch gezielte Synthese aus optisch reinen Ausgangsprodukten als reine Isomere hergestellt werden. Die einzelnen Isomeren zeigen unterschiedliche biologische Aktivitäten und bilden ebenso wie die Gemische einen wichtigen Gegenstand dieser Erfindung, d.h. die reinen Diastereomeren und die reinen Enantiomeren sind ebenso wie deren Gemische untereinander ein Bestandteil der vorliegenden Erfindung. Für den erfolgreichen Einsatz auf dem Agrargebiet genügt die Verwendung der Gemische, obgleich die reinen Isomeren in gewissen Fällen eine Verminderung der Dosis ermöglichen.

Es wurde nun überraschend gefunden, dass die neuen Wirkstoffe der Formel I bzw. Mittel, die diese Wirkstoffe enthalten, sich vor allem dadurch auszeichnen, dass sie gezielt in den Metabolismus der Pflanzen eingreifen. Dieser gezielte Eingriff in die physiologischen Vorgänge der Pflanzenentwicklung macht die Wirkstoffe der Formel I für verschiedene Zwecke verwendbar, insbesondere für solche, die mit der Ertragssteigerung bei Nutzpflanzen, der Ernteerleichterung und der Arbeitseinsparung bei Massnahmen an Pflanzenkulturen im Zusammenhang stehen.

Für die Wirkungsweise von Pflanzenwachstumsregulatoren gilt nach der bisherigen Erfahrung, dass ein Wirkstoff eine oder auch mehrere verschiedenartige Wirkungen auf Pflanzen ausüben kann. Die Wirkungen der Stoffe hängen im wesentlichen ab von dem Zeitpunkt der Anwendung, bezogen auf das Entwicklungsstadium des Samens oder der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in gewünschter Weise positiv beeinflussen.

Pflanzenwuchsregulierende Stoffe können zum Beispiel zur Hemmung des vegetativen Pflanzenwachstums eingesetzt werden. Eine derartige Wuchshemmung ist unter anderem bei Gräsern von wirtschaftlichem Interesse, denn dadurch kann z.B. die Häufigkeit der Grasschnitte in Ziergärten, Park- und Sportanlagen oder an Strassenrändern reduziert werden. Von Bedeutung ist auch die Hemmung des Wuchses von krautigen und holzigen Pflanzen an Strassenrändern und in der Nähe von Ueberlandleitungen oder ganz allgemein in Bereichen, in denen ein starker Bewuchs unerwünscht ist.

Wichtig ist auch die Anwendung von Wachstumsregulatoren zur Hemmung des Längenwachstums bei Getreide, denn durch eine Halmverkürzung wird die Gefahr des Umknickens ("Lagerns") der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten- und Fruchtbildung zugute kommen, während das vegetative Wachstum eingeschränkt wird.

Mit Wachstumsregulatoren lässt sich häufig auch eine Förderung des vegetativen Wachstums erzielen. Dies ist von grossem Nutzen, wenn die vegetativen Pflanzenteile geerntet werden. Eine Förderung des vegetativen Wachstums kann aber auch gleichzeitig zu einer Förderung des generativen Wachstums führen, so dass z.B. mehr oder grössere Früchte zur Ausbildung kommen.
Ertragssteigerungen können in manchen Fällen auch durch einen Eingriff in den pflanzlichen Stoffwechsel erreicht werden, ohne dass sich Aenderungen des vegetativen Wachstums bemerkbar machen. Wachstumsregulatoren können ferner eine Veränderung der Zusammensetzung der Pflanzen bewirken,so dass eine bessere Qualität der Ernteprodukte herbeigeführt wird. So ist es beispielsweise möglich, den Gehalt an Zucker in Zuckerrüben, Zuckerrohr, Ananas sowie Zitrusfrüchten zu erhöhen oder den Proteingehalt in Soja oder Getreide zu steigern.

Unter dem Einfluss von Wachstumsregulatoren kann es zur Ausbildung parthenokarper Früchte kommen. Ferner kann das Geschlecht der Blüten beeinflusst werden.

Mit Wachstumsregulatoren lässt sich auch die Produktion oder der Abfluss von sekundären Pflanzenstoffen positiv beeinflussen. Als Beispiel sei die Stimulierung des Latexflusses bei Gummibäumen genannt.

Während des Wachstums der Pflanze kann durch Einsatz von Wachstumsregulatoren auch die seitliche Verzweigung durch eine chemische Brechung der Apikaldominanz vermehrt werden. Daran besteht z.B. Interesse bei der Stecklingsvermehrung von Pflanzen. Es ist jedoch auch möglich, das Wachstum der Seitentriebe zu hemmen, z.B. um bei Tabakpflanzen nach der Dekapitierung die Ausbildung von Seitentrieben zu verhindern und damit das Blattwachstum zu fördern.

Unter dem Einfluss von Wachstumsregulatoren kann der Blattbestand von Pflanzen so gesteuert werden, dass ein Entblättern der Pflanzen zu einem gewünschten Zeitpunkt erreicht wird. Eine derartige Entlaubung ist von Interesse, um eine mechanische Beerntung z.B. bei Wein

oder Baumwolle, zu erleichtern oder um die Transpiration zu einem Zeitpunkt herabzusetzen, an dem die Pflanze verpflanzt werden soll. Durch Einsatz von Wachstumsregulatoren lässt sich der vorzeitige Fruchtfall verhindern. Es ist jedoch auch möglich, den Fruchtfall, - zum Beispiel bei Obst -, im Sinne einer chemischen Ausdünnung bis zu einem bestimmten Ausmass zu fördern. Wachstumsregulatoren können auch dazu dienen, um bei Kulturpflanzen zum Zeitpunkt der Ernte die zum Ablösen der Früchte erforderliche Kraft zu vermindern, so dass eine mechanische Beerntung der Pflanzen ermöglicht, beziehungsweise eine manuelle Beerntung erleichtert wird.

Mit Wachstumsregulatoren lässt sich ferner eine Beschleunigung oder auch eine Verzögerung der Reife des Erntegutes vor oder nach der Ernte erreichen. Dieses ist von besonderem Vorteil, weil sich dadurch eine optimale Anpassung an die Bedürfnisse des Marktes herbeiführen lässt. Weiterhin können Wachstumsregulatoren in manchen Fällen die Fruchtausfärbung verbessern. Darüberhinaus kann mit Hilfe von Wachstumsregulatoren auch eine zeitliche Konzentrierung der Reife erzielt werden. Damit werden Voraussetzungen dafür geschaffen, dass z.B. bei Tabak, Tomaten oder Kaffee, eine vollständige mechanische oder manuelle Beerntung in nur einem Arbeitsgang vorgenommen werden kann.

Durch die Anwendung von Wachstumsregulatoren kann auch die Samen- oder Knospenruhe der Pflanzen,also die endogene Jahresrhythmik, beeinflusst werden, so dass die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen.

Mit Wachstumsregulatoren kann auch erreicht werden, dass der Austrieb von Knospen oder die Keimung von Samen verzögert wird, z.B. um in frostgefährdeten Gebieten eine Schädigung durch Spätfröste zu vermeiden. Andererseits gelingt es, das Wurzelwachstum zu und/oder die Ausbildung von Sprösslingen zu stimulieren, so dass das Wachstum auf eine kürzere Zeitdauer beschränkt werden kann.

Wachstumsregulatoren können auch eine Halophilie bei den Kulturpflanzen erzeugen. Damit werden die Voraussetzungen dafür geschaffen, dass eine Kultivierung von Pflanzen auf salzhaltigen Böden durchgeführt werden kann.

Mit Wachstumsregulatoren kann auch eine Frost- und Trockenresistenz bei Pflanzen induziert werden.

Unter dem Einfluss von Wachstumsregulatoren kann das Altern (die Seneszenz) von Pflanzen oder Pflanzenteilen gehemmt respektive verzögert werden. Eine solche Wirkung kann von hohem wirtschaftlichem Interesse sein, dadurch, dass bei behandelten Pflanzenteilen oder ganzen Pflanzen wie Obst, Beeren, Gemüse, Salat oder Zierpflanzen deren Lagerfähigkeit nach der Ernte verbessert oder verlängert werden kann. Ebenso kann durch Behandlung von Kulturpflanzen über eine Verlängerung der Phase photosynthetischer Aktivität eine beachtliche Ertragssteigerung erzielt werden.

Ein weiteres wichtiges Anwendungsgebiet für Wuchshemmer ist deren Einsatz zur Hemmung eines übermässigen Wachstums bei tropischen Bodenbeckungspflanzen, den sogenannten Cover crops. In tropischen und subtropischen Monokulturen, wie z.B. in Palmplantagen, Baumwoll-, Maisfeldern usw. werden neben den eigentlichen Kulturpflanzen oftmals Bodenbedeckungspflanzen, insbesondere Leguminosenarten angepflanzt, die zur Erhaltung oder Steigerung der Bodenqualität (Verhinderung der Austrocknung, Versorgung mit Stickstoff) und zur Verhinderung von Erosion (Abtragung durch Wind und Wasser) dienen. Durch Applikation der erfindungsgemässen Wirkstoffe kann nunmehr das Wachstum dieser Cover crops kontrolliert und somit die Wuchshöhe dieser Bodenbedeckungspflanzen auf einem niedrigen Niveau gehalten werden, so dass ein gesundes Gedeihen der Kulturpflanzen und die Aufrechterhaltung einer günstigen Bodenbeschaffenheit gewährleistet ist.

Weiter hat es sich überraschenderweise gezeigt, dass die Aktivsubstanzen der Formel I bzw. entsprechende Mittel, ausser vorteilhaften wuchsregulierenden Eigenschaften zusätzlich ein für praktische Bedürfnisse sehr günstiges Mikrobizidspektrum aufweisen. Deshalb liegt ein weiteres Einsatzgebiet von Verbindungen der Formel I in der Bekämpfung von schädlichen Mikroorganismen, vor allem von phytopathogegen Pilzen. So besitzen die Verbindungen der Formel I eine für praktische Bedürfnisse sehr günstige kurative, präventive und systemische Wirkung zum Schutz von Pflanzen, insbesondere Kulturpflanzen, ohne diese nachteilig zu beeinflussen.

Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe sind gegen die den folgenden Klassen angehörenden phytopathogenen Pilze wirksam: Ascomyceten (z.B. Venturia, Podosphaera, Erysiphe, Monilinia, Uncinula); Basidiomyceten (z.B. die Gattungen Hemileia, Rhizoctonia, Puccinia); Fungi imperfecti (z.B. Bortrytis, Helminthosporium, Fusarium, Septoria, Cercospora und Alternaria). Ueberdies wirken die Verbindungen der Formel I systemisch. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden. Die erfindungsgemässen Wirkstoffe zeichnen sich durch besonders gute Pflanzenverträglichkeit aus.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen.

Darüberhinaus schliesst die vorliegende Erfindung auch die Herstellung agrochemischer Mittel ein, die gekennzeichnet ist durch das innige Vermischen der Aktivsubstanz mit einem oder mehrereb hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkulturen für die hierin offenbarten Indikationsgebiete gelten im Rahmen dieser Erfindung beispielsweise folgende Pflanzenarten: Getreide: (Weizen, Gerste, Roggen, Hafer, Reis, Sorhum und Verwandte); Rüben: (Zucker- und Futterrüben); Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer):oder Pflanzen wie Mais, Tabak, Nüsse, Kaffee, Zuckerrohr, Tee, Weintrauben, Hopfen, Bananen- und Naturkautschukgewächse. Pflanzen seien im Rahmen vorliegender Erfindung aber auch alle Arten von sonstigen Grünbewachsungen, seien es Zierpflanzen (Compositen), Grasflächen, Böschungen oder allgemeine niedrige Bodenbedeckungen (cover crops), die einer Erosion oder Austrocknung des Bodens entgegenwirken oder Bodenbedeckungen wie sie in Baum- und Staudenkulturen (Obstplantagen, Hopfenkulturen, Maisfeldern, Weingärten usw.) erwünscht sind.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein, Es können aber auch selektive Herbizide, Insektizide, Fungizide, Bakterizide, Nematizide, Molluskizide oder

Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Anzahl der Applikationen richten sich dabei nach dem Befallsdruck für den entsprechenden Erreger (Pilzsorte) oder der Art der Wachstumsbeeinflussung. Die Wirkstoffe der Formel I können aber auch über den Erdboden durch das Wurzelwerk in die Pflanzen gelangen (systemische Wirkung), indem man den Standort der Pflanzen mit einer flüssigen Zubereitung tränkt oder die Substanzen in fester Form in den Boden einbringt, z.B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüberhinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie

Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 10 g bis 5 kg Aktivsubstanz (AS) je ha; bevorzugt 100g bis 2 kg AS/ha, insbesondere bei 200 g bis 600 g AS/ha.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegegenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierhier gehören auch die Salze der Schwefelsäureester und Sulfonsäuren von Fettalkohol-Ethylenoxyd-Addukten. Die sulfonierten Benzimidazolderivate enthalten, vorzugsweise 2-Sulfonsäuregruppen und einen Fettsäurerest mit 8-22 C-Atomen. Alkylarylsulfonate sind z.B. die

Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglycolether, Polypropylen-Polyethylenoxydaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als

Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ringwood New Jersey, 1980
Sisely and Wood, "Encyclopedia of Surface Active Agents", Chemical Publishing Co., Inc. New York, 1980.

Die agrochemischen Zubereitungen enthalten in der Regel 0,1 bis 99%, insbesondere 0,1 bis 95% Wirkstoff der Formel I, 99,9 bis 1%, insbesondere 99,8 bis 5% eines festen oder flüssigen Zusatzstoffes und 0 bis 25%, insbesondere 0,1 bis 25% eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe zu beschränken. Temperaturen sind in Celsiusgraden angegeben. Prozente und Teile beziehen sich auf das Gewicht. RT bedeutet Raumtemperatur, h steht für Stunde, min für Minute, DMSO für Dimethylsulfoxid, THF für Tetrahydrofuran, DMF für Dimethylformamid.

Herstellungsbeispiele

Beispiel 1: Herstellung von

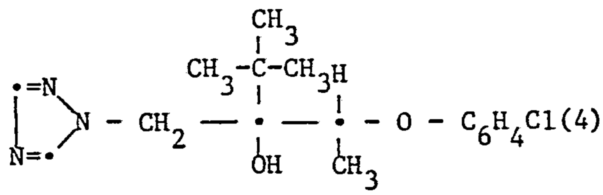

1-[1H-1,2,4-triazolyl]-2-t-butyl-2-hydroxy-3-[4-chlorphenoxy]-butan

a) Herstellung der Ausgangsprodukte:

1) 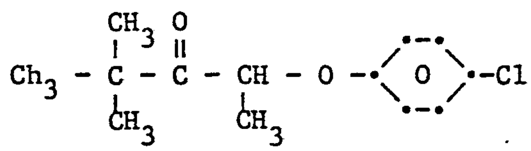

2,2-Dimethyl-4-[4-chlorphenoxy]-pentan-3-on

14,2 g NaH (50%ig in Oel-Dispersion) werden durch Dekantieren mit Hexan gewaschen und 30 ml Tetrahydrofuran zugegeben. 67,8 g 2,2-Dimethyl-4-[4-Chlorphenoxy]-butan-3-on und 57,6 g Methyljodid werden in 180 ml THF gelöst und bei 40-50° zur NaH-Suspension getropft. Nach beendeter Wasserstoffentwicklung wird noch 2 h nachgerührt, das überschüssige NaH mit Eisessig zerstört und das Reaktionsgemisch dann auf Eiswasser gegeben. Das Produkt wird dann mit Diethylether extrahiert, die organische Phase mit Sole gewaschen und über Natriumsulfat getrocknet. Rohausbeute 72 g, Gehalt 98% (IGC). Oel.

b) Herstellung des Zwischenproduktes

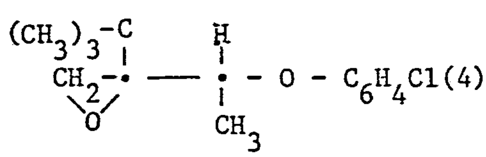

1-t-Butyl-1-[1-(4-chlorphenoxy)ethyl]-oxiran

20,3 g 50%ige Kaliumhydroxidlösung, 2 g Tetrabutylammoniumbromid, 12,4 g Trimethylsulfoxoniumjodid und 10,9 g 2,2-Dimethyl-4-(4-Chlor-

phenoxy)-pentan-3-on (Rohprodukt) werden zusammengegeben und 8 h auf 100° erhitzt. Die Phasen werden getrennt, die organische Phase mit Wasser gewaschen, über $Na_2SO_4$ getrocknet und eingedampft. 21,5 g eines gelben Oeles werden erhalten. Gehalt: ca. 60% Produkt, ca. 30% Edukt, ca. 10% nicht identifizierte Nebenprodukte (GC). Dieses Produkt wird ohne weitere Reinigung für die nächste Umsetzung verwendet.

c) Herstellung des Endproduktes:

21 g des vorgehend beschriebenen Gemisches, 5,7 g 1,2,4-Triazol und 1 g $LiOH \cdot H_2O$ werden in 100 ml DMSO gelöst und über Nacht auf 90° erhitzt. Das Reaktionsgemisch wird auf Eis/Wasser gegeben und mit Essigsäureethylester extrahiert. Die Esterphase wird mit Wasser und Sole gewaschen, über $Na_2SO_4$ getrocknet und eingedampft. Das anfallende Oel wird mit Ether verrührt, Kristallisation des 1-[4H-1,2,4-Triazolyl]-2-t-butyl-2-hydroxy-3-[4-chlorphenoxy]-butan-derivates, das abfiltriert wird. Das Filtrat wird eingedampft und mit Ether/Hexan das Produkt kristallisiert. Ausbeute 7,8 g des 1-[1H-1,2,4-Triazolyl]-2-t.-butyl-2-hydroxy-3-[4-chlorphenoxy]-butans. Smp. 108-109°.

| | | | | |
|---|---|---|---|---|
| Ber. | C 59.3% | H 6,85% | N 12,98% | Cl 10,95% |
| Gef. | C 59,1% | H 6,6% | N 12,9% | Cl 11,0%. |

Beispiel 2: Herstellung von

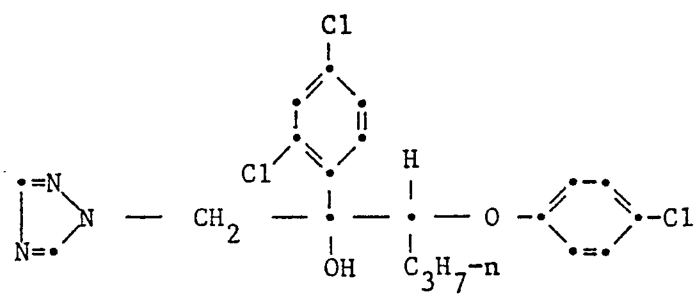

1-[1H-1,2,4-Triazolyl]-2-[2,4-dichlorphenyl]-2-hydroxy-3-(4-chlorphenoxy)-hexan

a) Herstellung des Ausgangsproduktes

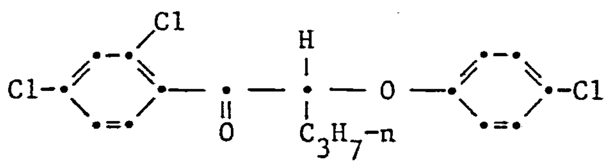

1-[2,4-Dichlorphenyl]-2-[4-chlorphenoxy]-pentanon-1

Eine Mischung von 129 g p-Chlorphenol und 138 g Kaliumcarbonat wird in 100 ml Aceton 3 h unter Rühren und unter Rückfluss erhitzt. Anschliessend lässt man bei RT und weiterem intensiven Rühren eine Lösung von 310 g α-Brom-2,4-dichlorvalerophenon in 500 ml Aceton zutropfen und rührt das Reaktionsgemisch weitere 10 h unter Rückfluss. Dann werden die organischen Salze abfiltriert, das Filtrat im Vakuum eingeengt, mit Wasser versetzt und mit Ethylacetat extrahiert, getrocknet, filtriert und eingedampft. Die organische Phase liefert ein braunes Oel, das nach Destillation im Hochvakuum 288 g von 1-[2,4-Dichlorphenyl]-2-[4-chlorphenoxy]-pentanon-1 als gelbes, viskoses Oel ergibt. Sdp.160-168°/0,001 mbar.

b) Herstellung des Zwischenproduktes

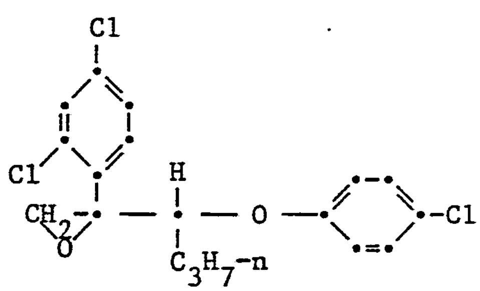

2-[2,4-Dichlorphenyl]-2-[2-(4-chlorphenoxy)-butyl]-oxiran

3,4 g 55%iges Natriumhydrid wird in 90 ml absolutes DMSO eingetragen, unter Rühren mit 17,8 g Trimethylsulfoxoniumjodid versetzt, 2 h bei RT gerührt und anschliessend mit einer Lösung von 25 g 1-[2,4-Dichlorphenyl]-2-[4-chlorphenoxy]-pentanon-1 in 80 ml absolutem THF versetzt. Die Lösung wird 2 h bei ca. 55-60° gerührt und dann auf eiskalte Solelösung gegossen. Die Extraktion mit Diethylether/Ethylacetat

(1:1) liefert 24,5 g rohes 2-[2,4-Dichlorphenyl]-2-[2-(4-chlorphenoxy)-butyl]-oxiran als gelbes Oel.

c) Herstellung des Endproduktes:

Zu einer Lösung von 91,4 g 1H-1,2,4-Triazol und 20 g Kalium-t-butoxylat in 850 ml absolutem DMF gibt man eine Lösung von 197 g 2-[2,4-Dichlorphenyl]-2-[2-(4-chlorphenoxy)-butyl]-oxiran in 850 ml DMF und erwärmt das Gemisch 15 h auf 95°. Nach dem Abkühlen auf RT wird die Mischung auf Kochsalzlösung gegossen und mit Ethylacetat extrahiert. Die vereinigten Extrakte werden mit Wasser gewaschen, über Natriumsulfat getrocknet, filtriert und im Vakuum eingedampft. Der Rückstand kristallisiert beim Digerieren mit Diethylether und liefert 99 g 1-[1H-1,2,4-Triazolyl]-2-[2,4-dichlorphenyl]-2-hydroxy-3-(4-chlorphenoxy)-hexan. Smp. 144-145°.

Auf analoge Weise werden auch die nachfolgenden Produkte der Formel I sowie die genannten Zwischenprodukte hergestellt:

Tabelle 1: Verbindungen der Formel

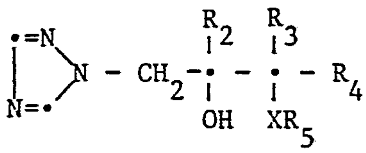

| Verb. Nr. | $R_2$ | $R_3$ | $R_4$ | $R_5$ | X | Physik. Konst. [°C] |
|---|---|---|---|---|---|---|
| 1.1 | $CH_3$ | $CH_3$ | $CH_3$ | $C_6H_4Cl(4)$ | O | Smp. 93-94° |
| 1.2 | $CH_3$ | $CH_3$ | $CH_3$ | $C_6H_3Cl_2(2,4)$ | O | Smp. 94-95° |
| 1.3 | $CH_3$ | $CH_3$ | $CH_3$ | $C_6H_3Cl(3,4)$ | O | Smp. 126-128° |
| 1.4 | $CH_3$ | $CH_3$ | $CH_3$ | 4-Biphenyl | O | Smp. 134-135° |
| 1.5 | $CH_3$ | $CH_3$ | $CH_3$ | $C_6H_4C_3H_7$-i(4) | O | Oel |
| 1.6 | $C_3H_7$-i | $CH_3$ | $CH_3$ | $C_6H_4Cl(4)$ | O | |
| 1.7 | $C_3H_7$-i | $CH_3$ | $CH_3$ | $C_6H_4F(4)$ | O | |
| 1.8 | $C_3H_7$-i | $CH_3$ | $CH_3$ | $C_6H_5$ | O | |
| 1.9 | $C_3H_7$-i | $CH_3$ | $CH_3$ | 4-Biphenyl | O | |
| 1.10 | $C_3H_7$-i | $CH_3$ | $CH_3$ | $C_6H_3Cl_2(2,3)$ | O | |
| 1.11 | $C_3H_7$-i | $CH_3$ | $CH_3$ | $C_6H_4(OCF_3)(4)$ | O | |
| 1.12 | $C_3H_7$-i | $CH_3$ | $CH_3$ | $C_6H_4(SCF_3)(4)$ | O | |
| 1.13 | $C_3H_7$-i | $CH_3$ | $CH_3$ | $C_6H_4(OCHF_2)(4)$ | O | |
| 1.14 | $C_6H_3Cl_2(2,4)$ | H | $C_2H_5$ | $C_6H_4CH_3(4)$ | O | Smp. 98-100° |
| 1.15 | $C_6H_3Cl_2(2,4)$ | H | $C_2H_5$ | $C_6H_4Cl(4)$ | O | Smp. 132-133° |
| 1.16 | $C_6H_3Cl_2(2,4)$ | H | $C_2H_5$ | $C_6H_4F(4)$ | O | Smp. 139-141° |
| 1.17 | $C_6H_3Cl_2(2,4)$ | H | $C_2H_5$ | $C_6H_3(CH_3)_2(2,3)$ | O | Smp. 121-122° |
| 1.18 | $C_6H_3Cl_2(2,4)$ | H | $C_3H_7$-n | $C_6H_4Cl(4)$ | O | Smp. 144-145° |
| 1.19 | $C_6H_3Cl_2(2,4)$ | H | $C_2H_5$ | $C_6H_5$ | O | Smp. 155-156° |
| 1.20 | $C_6H_3Cl_2(2,4)$ | H | $C_2H_5$ | $C_6H_4Br(4)$ | O | Smp. 121-124° |
| 1.21 | $C_6H_3Cl,F(2,4)$ | H | $CH_3$ | $C_6H_4Cl(4)$ | O | Smp. 133-136° |
| 1.22 | $C_6H_3Cl,F(2,4)$ | H | $CH_3$ | $C_6H_4(CH_3)(4)$ | O | Smp. 116-119° |
| 1.23 | $C_6H_4Cl(4)$ | H | $C_3H_7$-n | $C_6H_4Cl(4)$ | O | |
| 1.24 | $C_6H_3Cl(4)$ | H | $C_3H_7$-n | $C_6H_4F(4)$ | O | |
| 1.25 | $C_4H_9$-t | H | $CH_3$ | $C_6H_4Cl(4)$ | O | Smp. 108-109° |

Tabelle 1: (Fortsetzung)

| Verb. Nr. | $R_2$ | $R_3$ | $R_4$ | $R_5$ | X | Physik. Konst. [°C] |
|---|---|---|---|---|---|---|
| 1.26 | $C_4H_9$-t | H | $CH_3$ | $C_6H_4F(4)$ | O | Smp. 78-82° |
| 1.27 | $C_4H_9$-t | H | $CH_3$ | $C_6H_4CH_3(4)$ | O | Smp. 136-138° |
| 1.28 | $C_4H_9$-t | H | $C_2H_5$ | $C_6H_4Cl(4)$ | O | Smp. 135-137° |
| 1.29 | $C_4H_9$-t | H | $CH_3$ | $C_6H_5$ | O | |
| 1.30 | $C_4H_9$-t | H | $CH_3$ | $C_6H_4-CF_3(4)$ | O | Smp. 123-124° |
| 1.31 | $C_4H_9$-t | H | $CH_3$ | $C_6H_4OCF_3(4)$ | O | |
| 1.32 | $C_4H_9$-t | H | $CH_3$ | $C_6H_3(CH_3)_2(2,3)$ | O | |
| 1.33 | $C_4H_9$-t | H | $CH_3$ | $C_6H_3Cl_2(2,3)$ | O | |
| 1.34 | $C_4H_9$-t | H | $CH_3$ | $C_6H_4Br(4)$ | O | |
| 1.35 | $C_4H_9$-t | H | $CH_3$ | $C_6H_4SCHF_2(4)$ | O | |
| 1.36 | $C_4H_9$-t | H | $CH_3$ | $C_6H_4OCHF_2(4)$ | O | |
| 1.37 | $C_4H_9$-t | H | $CH_3$ | $C_6H_4Cl(4)$ | S | |
| 1.38 | $C_4H_9$-t | H | $C_2H_5$ | $C_6H_4F(4)$ | O | |
| 1.39 | $C_4H_9$-t | H | $C_2H_5$ | $C_6H_4Br(4)$ | O | |
| 1.40 | $C_4H_9$-t | H | $C_2H_5$ | $C_6H_4CH_3(4)$ | O | |
| 1.41 | $C_4H_9$-t | H | $C_2H_5$ | $C_6H_4CF_3(4)$ | O | |
| 1.42 | $C_4H_9$-t | H | $C_2H_5$ | $C_6H_4OCF_3(4)$ | O | |
| 1.43 | $C_4H_9$-t | H | $C_2H_5$ | $C_6H_5$ | O | |
| 1.44 | $C_4H_9$-t | H | $C_3H_7$-n | $C_6H_4Cl(4)$ | O | |
| 1.45 | $C_4H_9$-t | H | $C_3H_7$-n | $C_6H_4F(4)$ | O | |
| 1.46 | $C_4H_9$-t | H | $C_3H_7$-n | $C_6H_3Cl_2(2,4)$ | O | |
| 1.47 | $C_4H_9$-t | H | $C_3H_7$-n | $C_6H_4CH_3(4)$ | O | |
| 1.48 | $C_4H_9$-t | H | $C_3H_7$-n | $C_6H_4CF_3(4)$ | O | |
| 1.49 | $C_4H_9$-t | H | $C_3H_7$-n | $C_6H_5$ | O | |
| 1.50 | $C_4H_9$-t | H | $C_4H_9$-n | $C_6H_4Cl(4)$ | O | |
| 1.51 | $C_4H_9$-t | H | $C_4H_9$-n | $C_6H_4F(4)$ | O | |
| 1.52 | $C_4H_9$-t | H | $C_4H_9$-n | $C_6H_4CF_3(4)$ | O | |
| 1.53 | $C_4H_9$-t | H | $C_4H_9$-n | $C_6H_4Br(4)$ | O | |
| 1.54 | $C_4H_9$-t | H | $C_4H_9$-n | $C_6H_4CH_3(4)$ | O | |

Tabelle 1: (Fortsetzung)

| Verb. Nr. | $R_2$ | $R_3$ | $R_4$ | $R_5$ | X | Physik. Konst. [°C] |
|---|---|---|---|---|---|---|
| 1.55 | $C_3H_7$-i | H | $CH_3$ | $C_6H_4Cl(4)$ | O | |
| 1.56 | $C_3H_7$-i | H | $C_2H_5$ | $C_6H_4Cl(4)$ | O | Smp.69-73° |
| 1.57 | $C_3H_7$-i | H | $C_3H_7$-n | $C_6H_4Cl(4)$ | O | |
| 1.58 | $C_3H_7$-i | H | $CH_3$ | $C_6H_4F(4)$ | O | |
| 1.59 | $C_3H_7$-i | H | $C_2H_5$ | $C_6H_4F(4)$ | O | |
| 1.60 | $C_3H_7$-i | H | $CH_3$ | $C_6H_4CF_3(4)$ | O | |
| 1.61 | $C_3H_7$-i | H | $C_2H_5$ | $C_6H_4CF_3(4)$ | O | |
| 1.62 | $C_3H_7$-i | H | $C_3H_7$-n | $C_6H_4CF_3(4)$ | O | |
| 1.63 | $C_3H_7$-i | H | $CH_3$ | $C_6H_4Br(4)$ | O | |
| 1.64 | $C_3H_7$-i | H | $CH_3$ | $C_6H_4F(4)$ | O | |
| 1.65 | $C_3H_7$-i | H | $CH_3$ | $C_6H_4CH_3(4)$ | O | |
| 1.66 | $C_4H_9$-t | H | $CH_3$ | $C_6H_4CF_3(3)$ | O | |
| 1.67 | $C_4H_9$-t | H | $C_3H_5$ | $C_6H_4CF_3(3)$ | O | |
| 1.68 | $C_4H_9$-t | H | $CH_3$ | $C_6H_4Cl(3)$ | O | |
| 1.69 | $C_4H_9$-t | H | $C_2H_5$ | $C_6H_4Cl(3)$ | O | |
| 1.70 | $C_4H_9$-t | H | $C_3H_7$-n | $C_6H_4Cl(3)$ | O | |
| 1.71 | $C_4H_9$-t | H | $CH_3$ | $C_6H_3Cl(4)CH_3(2)$ | O | |
| 1.72 | $C_4H_9$-t | H | $C_2H_5$ | $C_6H_3(CH_3)_2(2,3)$ | O | |
| 1.73 | $C_4H_9$-t | H | $C_2H_5$ | $C_6H_3Cl_2(2,3)$ | O | |
| 1.74 | $C_4H_9$-t | H | $C_3H_7$-i | $C_6H_4Cl(4)$ | O | |
| 1.75 | $C_4H_9$-t | H | $C_3H_7$-i | $C_6H_4F(4)$ | O | |
| 1.76 | $C_4H_9$-t | H | $C_3H_7$-i | $C_6H_4Br(4)$ | O | |
| 1.77 | $C_4H_9$-t | H | $C_3H_7$-i | $C_6H_4CH_3(4)$ | O | |
| 1.78 | $C_4H_9$-t | H | $C_3H_7$-1 | $C_6H_4CF_3(4)$ | O | |
| 1.79 | α-Naphthyl | H | $CH_3$ | $C_6H_4Cl(4)$ | O | |
| 1.80 | β-Naphthyl | H | $CH_3$ | $C_6H_3Cl_2(2,4)$ | O | |
| 1.81 | α-Naphthyl | H | H | $C_6H_3Cl_2(2,4)$ | O | |
| 1.82 | α-Naphthyl | H | H | $C_6H_3Cl_2(2,4)$ | O | |
| 1.83 | $C_2H_5$ | H | H | $C_6H_4Cl(4)$ | O | |
| 1.84 | $C_2H_5$ | H | $CH_3$ | $C_6H_3Cl_2(2,3)$ | O | |
| 1.85 | $C_2H_5$ | $CH_3$ | $CH_3$ | $C_6H_4(CH_3)(4)$ | O | |

Tabelle 1: (Fortsetzung)

| Verb. Nr. | $R_2$ | $R_3$ | $R_4$ | $R_5$ | X | Physik. Konst. [°C] |
|---|---|---|---|---|---|---|
| 1.86 | $C_6H_4Cl(4)$ | H | $C_3H_7$-n | $C_6H_5$ | O | |
| 1.87 | $C_6H_4Cl(4)$ | H | $C_3H_7$-n | $C_6H_4CF_3(4)$ | O | |
| 1.88 | $C_6H_3Cl_2(2,4)$ | H | $CH_3$ | $C_6H_5$ | O | Smp. 150-152° |
| 1.89 | $C_6H_3Cl_2(2,4)$ | H | $CH_3$ | $C_6H_4Cl(4)$ | O | Smp. 130-132° |
| 1.90 | $C_6H_3Cl_2(2,4)$ | H | $CH_3$ | $C_6H_4CF_3(4)$ | O | |
| 1.91 | $C_6H_3Cl_2(2,4)$ | H | $CH_3$ | $C_6H_4OCF_3(4)$ | O | |
| 1.92 | $C_6H_3Cl_2(2,4)$ | H | $CH_3$ | $C_6H_4F(4)$ | O | Smp. 121-124° |
| 1.93 | $C_6H_3Cl_2(2,4)$ | H | $C_2H_5$ | $C_6H_4CF_3(4)$ | O | |
| 1.94 | $C_6H_3Cl_2(2,4)$ | H | $C_3H_7$-n | $C_6H_4F(4)$ | O | |
| 1.95 | $C_6H_3Cl_2(2,4)$ | H | $C_3H_7$-n | $C_6H_4OCF_3(4)$ | O | |
| 1.96 | $C_6H_3Cl_2(2,4)$ | H | $C_4H_9$-n | $C_6H_4F(4)$ | O | Smp. 174-175° |
| 1.97 | $C_6H_3Cl_2(2,4)$ | H | $C_4H_9$-n | $C_6H_4Cl(4)$ | O | Smp. 150-151° |
| 1.98 | $C_6H_3Cl_2(2,4)$ | H | $C_3H_7$-i | $C_6H_4F(4)$ | O | |
| 1.99 | $C_6H_3Cl,F(2.4)$ | H | $CH_3$ | $C_6H_4F(4)$ | O | |
| 1.100 | $C_6H_3Cl,F(2,4)$ | H | $C_2H_5$ | $C_6H_4Cl(4)$ | O | |
| 1.101 | $C_6H_3Cl,F(2,4)$ | H | $C_2H_5$ | $C_6H_4F(4)$ | O | |
| 1.102 | $C_6H_3Cl,F(2,4)$ | H | $C_3H_7$ | $C_6H_4F(4)$ | O | |

Tabelle 2: Verbindungen der Formel

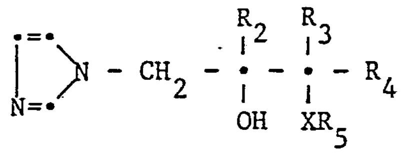

| Verb. Nr. | $R_2$ | $R_3$ | $R_4$ | $R_5$ | 0 | Physik. Konst. [°C] |
|---|---|---|---|---|---|---|
| 2.1 | $CH_3$ | $CH_3$ | $CH_3$ | $C_6H_4Cl(4)$ | 0 | |
| 2.2 | $CH_3$ | $CH_3$ | $CH_3$ | $C_6H_3Cl_2(2,4)$ | 0 | |
| 2.3 | $CH_3$ | $CH_3$ | $CH_3$ | $C_6H_3Cl_2(3,4)$ | 0 | |
| 2.4 | $CH_3$ | $CH_3$ | $CH_3$ | 4-Biphenyl | 0 | |
| 2.5 | $CH_3$ | $CH_3$ | $CH_3$ | $C_6H_3C_3H_7$-i(4) | 0 | |
| 2.6 | $CH_3$ | $CH_3$ | $CH_3$ | $CH_2CH_2C_6H_4(CH_3)(2)$ | 0 | |
| 2.7 | $C_3H_7$-i | $CH_3$ | $CH_3$ | $C_6H_4Cl(4)$ | 0 | |
| 2.8 | $C_3H_7$-i | $CH_3$ | $CH_3$ | $C_6H_4F(4)$ | 0 | |
| 2.9 | $C_3H_7$-i | $CH_3$ | $CH_3$ | $C_6H_5$ | 0 | |
| 2.10 | $C_3H_7$-i | $CH_3$ | $CH_3$ | 4-Biphenyl | 0 | |
| 2.11 | $C_3H_7$-1 | $CH_3$ | $CH_3$ | $C_6H_3Cl_3(2,3)$ | 0 | |
| 2.12 | $C_3H_7$-i | $CH_3$ | $CH_3$ | $C_6H_4(OCF_3)(4)$ | 0 | |
| 2.13 | $C_3H_7$-i | $CH_3$ | $CH_3$ | $C_6H_4(SCF_3)(4)$ | 0 | |
| 2.14 | $C_3H_7$-i | $CH_3$ | $CH_3$ | $C_6H_4(OCHF_2)(4)$ | 0 | |
| 2.15 | $C_3H_7$-i | $CH_3$ | $CH_3$ | $C_6H_4CN(4)$ | 0 | |
| 2.16 | $C_6H_3Cl_2(2,4)$ | $C_3H_7$-n | H | $C_6H_4Cl(4)$ | 0 | |
| 2.17 | $C_6H_3Cl_2(2,4)$ | $C_3H_7$-n | H | $C_6H_4F(4)$ | 0 | |
| 2.18 | $C_6H_3Cl_2(2,4)$ | $CH_3$ | H | $C_6H_4CF_3(4)$ | 0 | |
| 2.19 | $C_4H_9$-t | H | $CH_3$ | $C_6H_4Cl(4)$ | 0 | Smp.162-163° |
| 2.20 | $C_4H_9$-t | H | $C_2H_5$ | $C_6H_4Cl(4)$ | 0 | |
| 2.21 | $C_4H_9$-t | H | $C_3H_7$-i | $C_6H_4Cl(4)$ | 0 | |
| 2.22 | $C_4H_9$-t | H | $CH_3$ | $C_6H_4Br(4)$ | 0 | |
| 2.23 | $C_4H_9$-t | H | $C_2H_5$ | $C_6H_4Br(4)$ | 0 | |
| 2.24 | $C_4H_9$-t | H | $CH_3$ | $C_6H_4F$ (4) | 0 | Smp.177-178° |
| 2.25 | $C_4H_9$-t | H | $CH_3$ | $C_6H_4CH_3(4)$ | 0 | Smp.172-173° |
| 2.26 | $C_4H_9$-t | H | $C_2H_5$ | $C_6H_4F(4)$ | 0 | |

Tabelle 2: (Fortsetzung)

| Verb. Nr. | $R_2$ | $R_3$ | $R_4$ | $R_5$ | X | Physik. Konst. [°C] |
|---|---|---|---|---|---|---|
| 2.27 | $C_4H_9$-t | H | $C_2H_5$ | $C_6H_4CH_3(4)$ | O | |
| 2.28 | $C_4H_9$-t | H | $CH_3$ | $C_6H_4CF_3(4)$ | O | |
| 2.29 | $C_4H_9$-t | H | $C_2H_5$ | $C_6H_4CF_3(4)$ | O | |
| 2.30 | $C_3H_7$-i | H | $CH_3$ | $C_6H_4Cl(4)$ | O | |
| 2.31 | $C_3H_7$-i | H | $C_2H_5$ | $C_6H_4Cl(4)$ | O | |
| 2.32 | $C_3H_7$-i | H | $CH_3$ | $C_6H_4Br(4)$ | O | |
| 2.33 | $C_3H_7$-i | H | $CH_3$ | $C_6H_4F(4)$ | O | |
| 2.34 | $C_3H_7$-i | H | $CH_3$ | $C_6H_4CF_3(4)$ | O | |
| 2.35 | $C_4H_9$-t | H | $C_2H_5$ | $C_6H_4OCF_3(4)$ | O | |
| 2.36 | $C_4H_9$-t | H | $C_2H_5$ | $C_6H_5$ | O | |
| 2.37 | $C_4H_9$-t | H | $C_3H_7$-n | $C_6H_4Cl(4)$ | O | |
| 2.38 | $C_4H_9$-t | H | $C_3H_7$-n | $C_6H_4F(4)$ | O | |
| 2.39 | $C_4H_9$-t | H | $C_3H_7$-n | $C_6H_3Cl_2(2,4)$ | O | |
| 2.40 | $C_4H_9$-t | H | $C_3H_7$-n | $C_6H_4CH_3(4)$ | O | |
| 2.41 | $C_4H_9$-t | H | $C_3H_7$-n | $C_6H_4CF_3(4)$ | O | |
| 2.42 | $C_4H_9$-t | H | $C_3H_7$-n | $C_6H_5$ | O | |
| 2.43 | $C_4H_9$-t | H | $C_3H_7$-i | $C_6H_4CF_3(4)$ | O | |
| 2.44 | α-Naphthyl | H | $CH_3$ | $C_6H_4Cl(4)$ | O | |
| 2.45 | β-Naphthyl | H | $CH_3$ | $C_6H_3Cl_2(2,4)$ | O | |
| 2.46 | α-Naphthyl | H | H | $C_6H_3Cl_2(2,4)$ | O | |
| 2.47 | α-Naphthyl | H | H | $C_6H_3Cl_2(2,4)$ | S | |
| 2.48 | $C_2H_5$ | H | H | $C_6H_4Cl(4)$ | O | |
| 2.49 | $C_2H_5$ | H | $CH_3$ | $C_6H_3Cl_2(2,3)$ | O | |
| 2.50 | $C_2H_5$ | $CH_3$ | $CH_3$ | $C_6H_4(CH_3)(4)$ | O | |

Tabelle 3: Verbindungen der Formel

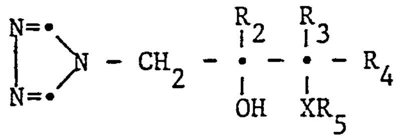

| Verb. Nr. | $R_2$ | $R_3$ | $R_4$ | $R_5$ | X | Physik. Konst. [°C] |
|---|---|---|---|---|---|---|
| 3.1 | $CH_3$ | H | $CH_3$ | 4-Benzyloxyphenyl | O | |
| 3.2 | $CH_3$ | H | $CH_3$ | $C_6H_4Cl(4)$ | S | |
| 3.3 | $CH_3$ | H | $CH_3$ | 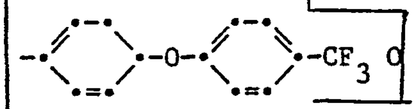 | O | |
| 3.4 | $CH_3$ | H | $CH_3$ | $C_6H_4C_2H_5(4)$ | O | |
| 3.5 | $CH_3$ | H | $CH_3$ | $C_6H_4C_3H_7$-i(4) | O | |
| 3.6 | $CH_3$ | $CH_3$ | $CH_3$ | $C_6H_4Cl(4)$ | O | |
| 3.7 | $CH_3$ | $CH_3$ | $CH_3$ | $C_6H_3Cl_2(2,4)$ | O | |
| 3.8 | $CH_3$ | $CH_3$ | $CH_3$ | $C_6H_3Cl_2(3,4)$ | O | |
| 3.9 | $CH_3$ | $CH_3$ | $CH_3$ | 4-Biphenyl | O | |
| 3.10 | $CH_3$ | $CH_3$ | $CH_3$ | $C_6H_3C_3H_7$-i(4) | O | |
| 3.11 | $CH_3$ | $CH_3$ | $CH_3$ | $C_6H_4(CH_3)(2)$ | O | |
| 3.12 | $C_3H_7$-i | $CH_3$ | $CH_3$ | $C_6H_4Cl(4)$ | O | |
| 3.13 | $C_3H_7$-i | $CH_3$ | $CH_3$ | $C_6H_4F(4)$ | O | |
| 3.14 | $C_3H_7$-i | $CH_3$ | $CH_3$ | $C_6H_5$ | O | |
| 3.15 | $C_3H_7$-i | $CH_3$ | $CH_3$ | 4-Biphenyl | O | |
| 3.16 | $C_6H_3Cl_2(2,4)$ | H | $CH_3$ | $C_6H_4Cl(4)$ | O | Smp.156-159 |
| 3.17 | $C_6H_3Cl_2(2,4)$ | H | $CH_3$ | $C_6H_3Cl_2(2,4)$ | O | |
| 3.18 | $C_6H_3Cl_2(2,4)$ | H | $CH_3$ | $C_6H_5$ | O | |
| 3.19 | $C_6H_5$ | H | $CH_3$ | $C_6H_4F(4)$ | O | |
| 3.20 | $C_4H_9$-t | H | $CH_3$ | $C_6H_4Cl(4)$ | O | fest |
| 3.21 | $C_4H_9$-t | H | $C_2H_5$ | $C_6H_4Cl(4)$ | O | |
| 3.22 | $C_4H_9$-t | H | $CH_3$ | $C_6H_4F(4)$ | O | Smp.197-195° |
| 3.23 | $C_4H_9$-t | H | $CH_3$ | $C_6H_4Br(4)$ | O | |
| 3.24 | $C_4H_9$-t | H | $CH_3$ | $C_6H_4CH_3(4)$ | O | |
| 3.25 | $C_4H_9$-t | H | $CH_3$ | $C_6H_5$ | O | |
| 3.26 | $C_4H_9$-t | H | $CH_3$ | $C_6H_4CF_3(4)$ | O | |

Tabelle 3: (Fortsetzung)

| Verb. Nr. | $R_2$ | $R_3$ | $R_4$ | $R_5$ | X | Physik. Konst. [°C] |
|---|---|---|---|---|---|---|
| 3.27 | $C_4H_9$-t | H | $CH_3$ | $C_6H_4CH_3(4)$ | O | |
| 3.28 | $C_3H_7$-i | $CH_3$ | $CH_3$ | $C_6H_3Cl_2(2,3)$ | O | |
| 3.29 | $C_3H_7$-i | $CH_3$ | $CH_3$ | $C_6H_4(OCF_3)(4)$ | O | |
| 3.30 | $C_3H_7$-i | $CH_3$ | $CH_3$ | $C_6H_4(SCF_3(4)$ | O | |
| 3.31 | $C_3H_7$-i | $CH_3$ | $CH_3$ | $C_6H_4(OCHF_2)(4)$ | O | |
| 3.32 | $C_3H_7$-i | $CH_3$ | $CH_2$ | $C_6H_4CN(4)$ | O | |
| 3.33 | $C_6H_3Cl_2(2,4)$ | H | $C_2H_5$ | $C_6H_2(CH_3)_2(3,5)Br(4)$ | O | Smp. 177-178° |
| 3.34 | $C_6H_3Cl_2(2,4)$ | H | $C_2H_5$ | $C_6H_4F(4)$ | O | |
| 3.35 | $C_6H_3Cl_2(2,4)$ | H | $C_2H_5$ | $C_6H_3(CH_3)_2(2,3)$ | O | |
| 3.36 | $C_6H_3Cl_2(2,4)$ | H | $C_3H_7$-n | $C_6H_4Cl(4)$ | O | |
| 3.37 | $C_6H_3Cl_2(2,4)$ | H | $C_2H_5$ | $C_6H_5$ | O | |
| 3.38 | $C_6H_3Cl_2(2,4)$ | H | $C_2H_5$ | $C_6H_4Br(4)$ | O | |
| 3.39 | $C_6H_3Cl,F(2,4)$ | H | $CH_3$ | $C_6H_4Cl(4)$ | O | |
| 3.40 | $C_6H_3Cl,F(2,4)$ | H | $CH_3$ | $C_6H_4(CH_3)(4)$ | O | |
| 3.41 | $C_4H_9$-t | H | $C_2H_5$ | $C_6H_4CF_3(3)$ | O | |
| 3.42 | $C_4H_9$-t | H | $CH_3$ | $C_6H_4Cl(3)$ | O | |
| 3.43 | $C_4H_9$-t | H | $C_2H_5$ | $C_6H_4Cl(3)$ | O | |
| 3.44 | $C_4H_9$-t | H | $C_3H_7$-n | $C_6H_4Cl(3)$ | O | |
| 3.45 | $C_4H_9$-t | H | $CH_3$ | $C_6H_3Cl(4)CH_3(2)$ | O | |
| 3.46 | $C_4H_9$-t | H | $C_2H_5$ | $C_6H_3(CH_3)_2(2,3)$ | O | |
| 3.47 | $C_4H_9$-t | H | $C_2H_5$ | $C_6H_3Cl_2(2,3)$ | O | |
| 3.48 | $C_4H_9$-t | H | $C_3H_7$-i | $C_6H_4Cl(4)$ | | |
| 3.49 | $C_4H_9$-t | H | $C_3H_7$-i | $C_6H_4F(4)$ | O | |
| 3.50 | $C_4H_9$-t | H | $C_3H_7$-i | $C_6H_4CH_3(4)$ | O | |
| 3.51 | $C_4H_9$-t | H | $C_3H_7$-i | $C_6H_4CH_3(4)$ | O | |
| 3.52 | $C_4H_9$-t | H | $C_3H_7$-i | $C_6H_4CF_3(4)$ | O | |
| 3.53 | α-Naphthyl | H | $CH_3$ | $C_6H_4Cl(4)$ | O | |
| 3.54 | β-Naphthyl | H | $CH_3$ | $C_6H_3Cl_2(2,4)$ | O | |
| 3.55 | α-Naphthyl | H | H | $C_6H_3Cl_2(2,4)$ | O | |

Tabelle 3: (Fortsetzung)

| Verb. Nr. | $R_2$ | $R_3$ | $R_4$ | $R_5$ | X | Physik. Konst. [°C] |
|---|---|---|---|---|---|---|
| 3.56 | α-Naphthyl | H | H | $C_6H_3Cl_2(2,4)$ | S | |
| 3.57 | $C_2H_5$ | H | H | $C_6H_4Cl(4)$ | O | |
| 3.58 | $C_2H_5$ | H | $CH_3$ | $C_6H_3Cl_2(2,3)$ | O | |
| 3.59 | $C_2H_5$ | $CH_3$ | $CH_3$ | $C_6H_4(CH_3)(4)$ | O | |

Tabelle 4: Zwischenprodukte der Formel II

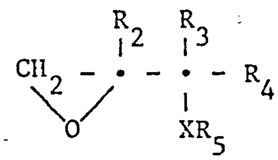

(II)

| Verb. Nr. | $R_2$ | $R_3$ | $R_4$ | $R_5$ | X | Physik. Konst.[°C] |
|---|---|---|---|---|---|---|
| 4.1 | $CH_3$ | $CH_3$ | $CH_3$ | $C_6H_4Cl(4)$ | O | Oel |
| 4.2 | $CH_3$ | $CH_3$ | $CH_3$ | $C_6H_3Cl_2(2,4)$ | O | Oel |
| 4.3 | $CH_3$ | $CH_3$ | $CH_3$ | $C_6H_3Cl_2(3,4)$ | O | Oel |
| 4.4 | $CH_3$ | $CH_3$ | $CH_3$ | 4-Biphenyl | O | Oel |
| 4.5 | $CH_3$ | $CH_3$ | $CH_3$ | $C_6H_3C_3H_7$-i(4) | O | Oel |
| 4.6 | $C_3H_7$-i | $CH_3$ | $CH_3$ | $C_6H_4Cl(4)$ | O | |
| 4.7 | $C_3H_7$-i | $CH_3$ | $CH_3$ | $C_6H_4F(4)$ | O | |
| 4.8 | $C_3H_7$-i | $CH_3$ | $CH_3$ | $C_6H_5$ | O | |
| 4.9 | $C_3H_7$-i | $CH_3$ | $CH_3$ | 4-Biphenyl | O | |
| 4.10 | $C_3H_7$-i | $CH_3$ | $CH_3$ | $C_6H_3Cl(2,3)$ | O | |
| 4.11 | $C_3H_7$-i | $CH_3$ | $CH_3$ | $C_6H_4(OCF_3)(4)$ | O | |
| 4.12 | $C_3H_7$-i | $CH_3$ | $CH_3$ | $C_6H_4(SCF_3)(4)$ | O | |
| 4.13 | $C_3H_7$-i | $CH_3$ | $CH_3$ | $C_6H_4(OCHF_2)(4)$ | O | |
| 4.14 | $C_3H_7$-i | $CH_3$ | $CH_3$ | $C_6H_4CN(4)$ | O | |
| 4.15 | $C_6H_3Cl_2(2,4)$ | H | $C_2H_5$ | $C_6H_4Cl(4)$ | O | Oel |
| 4.16 | $C_6H_3Cl_2(2,4)$ | H | $C_2H_5$ | $C_6H_4F(4)$ | O | Oel |
| 4.17 | $C_6H_3Cl_2(2,4)$ | H | $C_2H_5$ | $C_6H_3(CH_3)_2(2,3)$ | O | Oel |
| 4.18 | $C_6H_3Cl_2(2,4)$ | H | $C_3H_7$-n | $C_6H_4Cl(4)$ | O | Oel |
| 4.19 | $C_6H_3Cl_2(2,4)$ | H | $C_2H_5$ | $C_6H_5$ | O | Oel |
| 4.20 | $C_6H_3Cl_2(2,4)$ | H | $C_2H_5$ | $C_6H_4Br(4)$ | O | Oel |
| 4.21 | $C_6H_3Cl,F(2,4)$ | H | $CH_3$ | $C_6H_4Cl(4)$ | O | Oel |
| 4.22 | $C_6H_3Cl,F(2,4)$ | H | $CH_3$ | $C_6H_4(CH_3)(4)$ | O | Oel |
| 4.23 | $C_6H_4Cl(4)$ | H | $C_3H_7$-n | $C_6H_4Cl(4)$ | O | Oel |
| 4.24 | $C_6H_3Cl(4)$ | H | $C_3H_7$-n | $C_6H_4F(4)$ | O | Oel |
| 4.25 | $C_4H_5$-t | H | $CH_3$ | $C_6H_4Cl(4)$ | O | Oel |

Tabelle 4: (Fortsetzung)

| Verb. Nr. | $R_2$ | $R_3$ | $R_4$ | $R_5$ | X | Physik. Konst.[°C] |
|---|---|---|---|---|---|---|
| 4.26 | $C_4H_9$-t | H | $CH_3$ | $C_6H_4F(4)$ | O | Sdp.76-78°/ 0,01 Torr |
| 4.27 | $C_4H_9$-t | H | $CH_3$ | $C_6H_4CH_3(4)$ | O | Sdp.80-85°/ 0,05 Torr |
| 4.28 | $C_4H_9$-t | H | $C_2H_5$ | $C_6H_4Cl(4)$ | O | Sdp.90-95°/ 0,01 Torr |
| 4.29 | $C_4H_9$-t | H | $CH_3$ | $C_6H_5$ | O | |
| 4.30 | $C_4H_9$-t | H | $CH_3$ | $C_6H_4CF_3(4)$ | O | |
| 4.31 | $C_4H_9$-t | H | $CH_3$ | $C_6H_4OCF_3(4)$ | O | |
| 4.32 | $C_4H_9$-t | H | $CH_3$ | $C_6H_3(CH_3)_2(2,3)$ | O | |
| 4.33 | $C_4H_9$-t | H | $CH_3$ | $C_6H_3Cl_2(2,3)$ | O | |
| 4.34 | $C_4H_9$-t | H | $CH_3$ | $C_6H_4Br(4)$ | O | |
| 4.35 | $C_4H_9$-t | H | $CH_3$ | $C_6H_4SCHF_2(4)$ | O | |
| 4.36 | $C_4H_9$-t | H | $CH_3$ | $C_6H_4OCHF_2(4)$ | O | |
| 4.37 | $C_4H_9$-t | H | $CH_3$ | $C_6H_4Cl(4)$ | S | |
| 4.38 | $C_4H_9$-t | H | $C_2H_5$ | $C_6H_4F(4)$ | O | |
| 4.39 | $C_4H_9$-t | H | $C_2H_5$ | $C_6H_4Br(4)$ | O | |
| 4.40 | $C_4H_9$-t | H | $C_2H_5$ | $C_6H_4CH_3(4)$ | O | |
| 4.41 | $C_4H_9$-t | H | $C_2H_5$ | $C_6H_4CF_3(4)$ | O | |
| 4.42 | $C_4H_9$-t | H | $C_2H_5$ | $C_6H_4OCF_3(4)$ | O | |
| 4.43 | $C_4H_9$-t | H | $C_2H_5$ | $C_6H_5$ | O | |
| 4.44 | $C_4H_9$-t | H | $C_3H_7$-n | $C_6H_4Cl(4)$ | O | |
| 4.45 | $C_4H_9$-t | H | $C_3H_7$-n | $C_6H_4F(4)$ | O | |
| 4.46 | $C_4H_9$-t | H | $C_3H_7$-n | $C_6H_3Cl_2(2,4)$ | O | |
| 4.47 | $C_4H_9$-t | H | $C_3H_7$-n | $C_6H_4CH_3(4)$ | O | |
| 4.48 | $C_4H_9$-t | H | $C_3H_7$-n | $C_6H_4CF_3(4)$ | O | |
| 4.49 | $C_4H_9$-t | H | $C_3H_7$-n | $C_6H_5$ | O | |
| 4.50 | $C_4H_9$-t | H | $C_4H_9$-n | $C_6H_4Cl(4)$ | O | |
| 4.51 | $C_4H_9$-t | H | $C_4H_9$-n | $C_6H_4F(4)$ | O | |
| 4.52 | $C_4H_9$-t | H | $C_4H_9$-n | $C_6H_4CF_3(4)$ | O | |
| 4.53 | $C_4H_9$-t | H | $C_4H_9$-n | $C_6H_4Br(4)$ | O | |
| 4.54 | $C_4H_9$-t | H | $C_4H_9$-n | $C_6H_4CH_3(4)$ | O | |
| 4.55 | $C_3H_7$-i | H | $CH_3$ | $C_6H_4Cl(4)$ | O | |

0070798



Tabelle 4: (Fortsetzung)

| Verb. Nr. | $R_2$ | $R_3$ | $R_4$ | $R_5$ | X | Physik. Konst. [°C] |
|---|---|---|---|---|---|---|
| 4.56 | $C_3H_7$-i | H | $C_2H_5$ | $C_6H_4Cl(4)$ | O | Oel |
| 4.57 | $C_3H_7$-i | H | $C_3H_7$-n | $C_6H_4Cl(4)$ | O | |
| 4.58 | $C_3H_7$-i | H | $CH_3$ | $C_6H_4F(4)$ | O | |
| 4.59 | $C_3H_7$-i | H | $C_2H_5$ | $C_6H_4F(4)$ | O | |
| 4.60 | $C_3H_7$-i | H | $CH_3$ | $C_6H_4CF_3(4)$ | O | |
| 4.61 | $C_3H_7$-i | H | $C_2H_5$ | $C_6H_4CF_3(4)$ | O | |
| 4.62 | $C_3H_7$-i | H | $C_3H_7$-n | $C_6H_4CF_3(4)$ | O | |
| 4.63 | $C_3H_7$-i | H | $CH_3$ | $C_6H_4Br(4)$ | O | |
| 4.64 | $C_3H_7$-i | H | $CH_3$ | $C_6H_4F(4)$ | O | |
| 4.65 | $C_3H_7$-i | H | $CH_3$ | $C_6H_4CH_3(4)$ | O | |
| 4.66 | $C_4H_9$-t | H | $CH_3$ | $C_6H_4CF_3(3)$ | O | |
| 4.67 | $C_4H_9$-t | H | $C_2H_5$ | $C_6H_4CF_3(3)$ | O | |
| 4.68 | $C_4H_9$-t | H | $CH_3$ | $C_6H_4Cl(3)$ | O | |
| 4.69 | $C_4H_9$-t | H | $C_2H_5$ | $C_6H_4Cl(3)$ | O | |
| 4.70 | $C_4H_9$-t | H | $C_3H_7$-n | $C_6H_4Cl(3)$ | O | |
| 4.71 | $C_4H_9$-t | H | $CH_3$ | $C_6H_3Cl(4)CH_3(2)$ | O | |
| 4.72 | $C_4H_9$-t | H | $C_2H_5$ | $C_6H_3(CH_3)_2(2,3)$ | O | |
| 4.73 | $C_4H_9$-t | H | $C_2H_5$ | $C_6H_3Cl_2(2,3)$ | O | |
| 4.74 | $C_4H_9$-t | H | $C_3H_7$-i | $C_6H_4Cl(4)$ | O | |
| 4.75 | $C_4H_9$-t | H | $C_3H_7$-i | $C_6H_4F(4)$ | O | |
| 4.76 | $C_4H_9$-t | H | $C_3H_7$-i | $C_6H_4Br(4)$ | O | |
| 4.77 | $C_4H_9$-t | H | $C_3H_7$-i | $C_6H_4CH_3(4)$ | O | |
| 4.78 | $C_4H_9$-t | H | $C_3H_7$-i | $C_6H_4CF_3(4)$ | O | |
| 4.79 | α-Naphthyl | H | $CH_3$ | $C_6H_4Cl(4)$ | O | |
| 4.80 | β-Naphthyl | H | $CH_3$ | $C_6H_3Cl_2(2,4)$ | O | |
| 4.81 | α-Naphthyl | H | H | $C_6H_3Cl_2(2,4)$ | O | |
| 4.82 | α-Naphthyl | H | H | $C_6H_3Cl_2(2,4)$ | S | |
| 4.83 | $C_2H_5$ | H | H | $C_6H_4Cl(4)$ | O | |
| 4.84 | $C_2H_5$ | H | $CH_3$ | $C_6H_3Cl_2(2,3)$ | O | |
| 4.85 | $C_2H_5$ | $CH_3$ | $CH_3$ | $C_6H_4(CH_3)(4)$ | O | |

Tabelle 4: (Fortsetzung)

| Verb. Nr. | $R_2$ | $R_3$ | $R_4$ | $R_5$ | X | Physik. Konst. [°C] |
|---|---|---|---|---|---|---|
| 4.86 | $C_6H_4Cl(4)$ | H | $C_3H_7$-n | $C_6H_5$ | O | |
| 4.87 | $C_6H_4Cl(4)$ | H | $C_3H_7$-n | $C_6H_4CF_3(4)$ | O | |
| 4.88 | $C_6H_3Cl_2(2,4)$ | H | $CH_3$ | $C_6H_5$ | O | |
| 4.89 | $C_6H_3Cl_2(2,4)$ | H | $CH_3$ | $C_6H_4Cl(4)$ | O | |
| 4.90 | $C_6H_3Cl_2(2,4)$ | H | $CH_3$ | $C_6H_4CF_3(4)$ | O | |
| 4.91 | $C_6H_3Cl_2(2,4)$ | H | $CH_3$ | $C_6H_4OCF_3(4)$ | O | |
| 4.92 | $C_6H_3Cl_2(2,4)$ | H | $CH_3$ | $C_6H_4F(4)$ | O | |
| 4.93 | $C_6H_3Cl_2(2,4)$ | H | $C_2H_5$ | $C_6H_4CF_3(4)$ | O | |
| 4.94 | $C_6H_3Cl_2(2,4)$ | H | $C_3H_7$-n | $C_6H_4F(4)$ | O | |
| 4.95 | $C_6H_3Cl_2(2,4)$ | H | $C_3H_7$-n | $C_6H_4OCF_3(4)$ | O | |
| 4.96 | $C_6H_3Cl_2(2,4)$ | H | $C_4H_9$-n | $C_6H_4F(4)$ | O | |
| 4.97 | $C_6H_3Cl_2(2,4)$ | H | $C_4H_9$-n | $C_6H_4Cl(4)$ | O | |
| 4.98 | $C_6H_3Cl_2(2,4)$ | H | $C_3H_7$-i | $C_6H_4F(4)$ | O | |
| 4.99 | $C_6H_3Cl,F(2.4)$ | H | $VH_3$ | $C_6H_4F(4)$ | O | |
| 4.100 | $C_6H_3Cl,F(2,4)$ | H | $C_2H_5$ | $C_6H_4Cl(4)$ | O | |
| 4.101 | $C_6H_3Cl,F(2,4)$ | H | $C_2H_5$ | $C_6H_4F(4)$ | O | |
| 4.102 | $C_6H_3Cl,F(2,4)$ | H | $C_3H_7$ | $C_6H_4F(4)$ | O | |

Formulierungsbeispiele für flüssige Wirkstoffe der Formel I

(% = Gewichtsprozent)

| 2. Emulsions-Konzentrate | a) | b) | c) |
|---|---|---|---|
| Wirkstoff aus den Tabellen 1 bis 3 | 25% | 40% | 50% |
| Ca-Dodecylbenzolsulfonat | 5% | 8% | 6% |
| Ricinusöl-polyethylenglykolether (36 Mol Ethylenoxid) | 5% | - | - |
| Tributylphenoyl-polyethylenglykol-ehter (30 Mol Ethylenoxid) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

| 3. Lösungen | a) | b) | c) | d) |
|---|---|---|---|---|
| Wirkstoff aus den Tabellen 1 bis 3 | 80% | 10% | 5% | 95% |
| Ethylenglykol-monomethyl-ether | 20% | - | - | - |
| Polyethylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

(MG = Molekulargewicht)

Die Lösungen sind zur Anwendung in Form kleinster Tropfen geeignet.

| 4. Granulate | a) | b) |
|---|---|---|
| Wirkstoff aus den Tabellen 1 bis 3 | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| 5. Stäubemittel | a) | b) |
|---|---|---|
| Wirkstoff aus den Tabellen 1 bis 3 | 2% | 5% |
| Hochdisperse Kieselsäure | 1% | 5% |
| Talkum | 97% | - |
| Kaolin | - | 90% |

Durch inniges Vermischen auf Trägerstoffe mit dem Wirkstoff erhält man gebrauchsfertige Stäubemittel.

Formulierungsbeispiele für feste Wirkstoffe der Formel I

(% = Gewichtsprozent)

| 6. Spritzpulver | a) | b) | c) |
|---|---|---|---|
| Wirkstoff aus den Tabellen 1 bis 3 | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyethylenglykolether (7-8 Mol Ethylenoxid) | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

7. Emulsions-Konzentrat

| | |
|---|---|
| Wirkstoff aus den Tabellen 1 bis 3 | 10% |
| Octylphenolpolyethylenglykolether (4-5 Mol Ethylenoxid) | 3% |
| Ca-Dodecylbenzolsulfonat | 3% |
| Ricinusölpolyglykolether (35 Mol Ethylenoxid) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| 8. Stäubemittel | a) | b) |
|---|---|---|
| Wirkstoff aus den Tabellen 1 bis 3 | 5% | 8% |
| Talkum | 95% | - |
| Kaolin | - | 92% |

Man erhält anwendungsfertige Stäubemittel, indem der Wirkstoff mit den Träger vermischt und auf einer geeigneten Mühle vermahlen wird.

9. Extruder Granulat

| | |
|---|---|
| Wirkstoff aus den Tabellen 1 bis 3 | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

10. Umhüllungs-Granulat

| | |
|---|---|
| Wirkstoff aus den Tabellen 1 bis 3 | 3% |
| Polyethylenglykol (M G 200) | 3% |
| Kaolin | 94% |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

11. Suspensions-Konzentrat

| | |
|---|---|
| Wirkstoff aus den Tabellen 1 bis 3 | 40% |
| Ethylenglykol | 10% |
| Nonylphenolpolyethylenglykolether (15 Mol Ethylenoxid) | 6% |
| N-Lingninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele:

Beispiel 12: Wirkung gegen Puccinia graminis auf Weizen

a) Residual-protektive Wirkung

Weizenpflanzen wurden 6 Tage nach der Aussaat mit einer aus Spritzpulver des Wirkstoffes hergestellten Spritzbrühe (0,06% Aktivsubstanz) besprüht. Nach 24 Stunden wurden die behandelten Pflanzen mit einer Uredosporensuspension des Pilzes infiziert. Nach einer Inkubation während 48 Stunden bei 95-100% relativer Luftfeuchtigkeit

und ca. 20°C wurden die infizierten Pflanzen in einem Gewächshaus bei ca. 22°C aufgestellt. Die Beurteilung der Rotpustelnentwicklung erfolgte 12 Tage nach der Infektion.

b) Systemische Wirkung

Zu Weizenpflanzen wurde 5 Tage nach der Aussaat eine aus Spritzpulver des Wirkstoffes hergestellte Spritzbrühe gegossen (0,006% Aktivsubstanz bezogen auf das Bodenvolumen). Nach 48 Stunden wurden die behandelten Pflanzen mit einer Ureidosporensuspension des Pilzes infiziert. Nach einer Inkubation während 48 Stunden bei 95-100% relativer Luftfeuchtigkeit und ca. 20°C wurden die infizierten Pflanzen in einem Gewächshaus bei ca. 22°C aufgestellt. Die Beurteilung der Rostpustelnentwicklung erfolgte 12 Tage nach der Infektion.

Verbindungen aus den Tabellen 1 bis 3 zeigten gegen Puccinia-Pilze eine gute Wirkung. Unbehandelte aber infizierte Kontrollpflanzen zeigten einen Puccinia-Befall von 100%. Unter anderem hemmten die Verbidnungen 1.1 bis 1.5, 1.15 bis 1.20, 1.25 bis 1.28, 3.20, 3.22 und 3.33 dem Puccinia-Befall auf 0 bis 15%.

Beispiel 13: Wirkung gegen Cercospora arachidicola auf Erdnusspflanzen

a) Residual-protektive Wirkung

10-15 cm hohe Erdnusspflanzen wurden mit einer aus Spritzpulver der Wirksubstanz hergestellten Spritzbrühe (0,02% Aktivsubstanz) besprüht und 48 Stunden später mit einer Konidiensuspension des Pilzes infiziert. Die infizierten Pflanzen wurden während 72 Stunden bei ca. 21°C und hoher Luftfeuchtigkeit inkubiert und anschliessend bis zum Auftraten der typischen Blattflecken in einem Gewächshaus aufgestellt. Die Beurteilung der fungiziden Wirkung erfolgte 12 Tage nach der Infektion basierend auf Anzahl und Grösse der auftretenden Flecken.

b) Systemische Wirkung

Zu 10-15 cm hohen Erdnusspflanzen wurde ine aus Spritzpulver des Wirkstoffes hergestellte Spritzbrühe gegossen (0,06% Aktivsubstanz bezogen auf das Erdvolumen). Nach 48 Stunden wurden die behandelten Pflanzen mit einer Konidiensuspension des Pilzes infiziert und 72 Stunden bei ca. 21°C und hoher Luftfeuchtigkeit inkubiert. Anschliessend wurden die Pflanzen im Gewächshaus aufgestellt und nach 11 Tagen der Pilzbefall beurteilt.

Im Vergleich zu unbehandelten, aber infizierten Kontrollpflanzen (Anzahl und Grösse der Flecken = 100%), zeigten Erdnusspflanzen, die mit Wirkstoffen aus den Tabellen 1 bis 3 behandelt wurden, einen stark reduzierten Cercospora-Befall. So verhinderten die Verbindungen 1.1 bis 1.5, insbesondere 1.15 bis 1.20, aber auch 1.25 bis 1.28, 3.20, 3.22 und 3.33 in obigen Versuchen das Auftreten von Flekcne fast vollständig (0-10%).

Beispiel 14: Wirkung gegen Erysiphae graminis auf Gerste

a) Residual-protektive Wirkung

Ca. 8 cm hohe Gerstenpflanzen wurden mit einer aus Spritzpulver des Wirkstoffes hergestellten Spritzbrühe (0,02% Aktivsubstanz) besprüht. Nach 3-4 Stunden wurden die behandelten Pflanzen mit Konidien des Pilzes bestäubt. Die infizierten Gerstenpfkanzen wurden in einem Gewächshaus bei ca. 22°C aufgestellt und der Pilzbefall nach 10 Tagen beurteilt.

b) Systemische Wirkung

Zu ca. 8 cm hohen Gerstenpflanzen wurde eine aus Spritzpulver des Wirkstoffes hergestellte Spritzbrühe gegossen (0,006% Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden

in einem Gewächshaus bei ca. 22°C aufgestellt und der Pilzbefall nach 10 Tagen beurteilt.

Verbindungen der Formel I zeigten eine gute Wirkung gegen Erysiphe-Pilze. Unbehandelte, aber infizierte Kontrollpflanzen zeigten einen Erysiphe-Befall von 100%. Unter anderen Verbindungen aus den Tabellen 1 bis 3 hemmten die Verbindungen Nr. 1.1 bis 1.5, insbesondere 1.15 bis 1.20, aber auch 1.25 bis 1.28 und 3.20 den Pilzbefall bei Gerste auf 0 bis 15%.

Beispiel 15: Residual-protektive Wirkung gegen Venturia inaequalis auf Apfeltrieben

Apfelstecklinge mit 10-20 cm langen Frischtrieben wurden mit einer aus Spritzpulver des Wirkstoffes hergestellten Spritzbrühe (0,06% Aktivsubstanz) besprüht. Nach 24 Stunden wurden die behandelten Pflanzen mit einer Konidiensuspension des Pilzes infiziert. Die Pflanzen wurden dann während 5 Tagen bei 90-100% relativer Luftfeuchtigkeit inkubiert und während 10 weiteren Tagen in einem Gewächshaus bei 20-24°C aufgestellt. Der Schorfbefall wurde 15 Tage nach der Infektion beurteilt. Verbindungen 1.1 bis 1.5, insbesondere 1.15 bis 1.20, aber auch 1.25 bis 1.28 und 3.20 und andere hemmten den Krankheitsbefall auf weniger als 10%. Unbehandelte aber infizierte Triebe zeigten einen 100%igen Venturia-Befall.

Beispiel 16: Wirkung gegen Botrytis cinerea auf Bohnen Residual protektive Wirkung

Ca. 10 cm hohe Bohnen-Pflanzen wurden mir einer aus Spritzpulver des Wirkstoffes hergestellten Spritzbrühe (0,02% Aktivsubstanz) besprüht. Nach 48 Stunden wurden die behandelten Pflanzen mit einer Konidiensuspension des Pilzes infiziert. Nach einer Inkubation der infizierten Pflanzen während 3 Tagen bei 95-100% relativer Luftfeuchtigkeit und 21°C erfolgte die Beurteilung des Pilzbefalls. Die Verbindungen

aus den Tabellen 1 bis 3 hemmten in vielen Fällen die Pilzinfektion sehr stark. Bei einer Konzentration von 0,02% erwiesen sich z.B. die Verbindungen nr. 1.15, 1.16, 1.17, 1.18, 1.19 und 1.20 als voll wirksam (Krankheitsbefall 0 bis 5%). Der Botrytis-Befall unbehandelter aber infizierter Bohnenpflanzen betrug 100%.

Beispiel 17: Wuchshemmung bei Getreide

In Kunststofftöpfen mit sterilisierter Erde wurden die Getreidearten Hordeum vulgare (Sommergerste) und Secale (Sommerroggen) im Gewächshaus angesät und nach Bedarf bewässert. Die Sprösslinge wurden ca. 21 Tage nach der Aussaat mit der wässrigen Spritzbrühe eines Wirkstoffes der Formel I besprüht. Die Wirkstoffmenge betrug umgerechnet 0,5 bzw. 2,5 kg Aktivsubstanz pro Hektar. 10 und 21 Tage nach Applikation wurde das Wachstum des Getreides beurteilt. Hierbei konnte festgestellt werden, dass Getreidepflanzen, die mit Wirkstoffen der Formel I behandelt worden waren, im Vergleich zu unbehandelten Kontrollpflanzen eine starke Wuchsreduktion aufweisen. Als besonders wirksam erwiesen sich Verbindungen aus den Tabellen 1 und 3. So reduzierten die Verbindungen Nr. 1.1 bis 1.5, insbesondere 1.15 bis 1.20, aber auch 1.25 bis 1.28 und 3.20 die Zuwachsrate auf weniger als 10%.

Beispiel 18: Wuchshemmung bei Gräsern

In Kunststoffschalen mit Erde-Torf-Sand-Gemisch (6:3:1) wurden die Gräser Lolium perenne, Poa pratensis, Festuca orina und Cynodon dactylon im Gewächshaus angesät und nach Bedarf bewässert. Die aufgelaufenen Gräser wurden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge betrug umgerechnet 0,5 bzw. 2,5 kg Aktivsubstanz pro Hektar. 10 und 21 Tage nach Applikation wurde das Wachstum der Gräser beurteilt, dabei zeigte es sich, dass die erfindungsgemässen

Wirkstoffe aus den Tabellen 1 bis 3 eine merkliche Wuchshemmung bewirkten. Besonders deutliche Wuchshemmung wurde mit den Verbindungen aus den Tabellen 1 und 3 bewirkt, so reduzierten die Verbindungen Nr. 1.1 bis 1.5, 1.15 bis 1.20, 1.25 bis 1.28, 3.20, 3.22 und 3.33 das Weiterwachsen fast vollständig (Zuwachsrate 0 bis 10%).

Beispiel 19: Ertragssteigerung an Sojabohnen

In Kunststoffbehältern mit einem Erde-Torf-Sandgemisch im Verhältnis 6:3:1 wurden Sojabohnen der Sorte "Hark" angesät und in eine Klimakammer gegeben. Durch optimale Temperaturwahl, Beleuchtung, Düngerzugabe und Bewässerung entwickelten sich die Pflanzen nach ca. 5 Wochen in dem 5-6 Trifola-Blattstadium. Zu diesem Zeitpunkt wurden die Pflanzen mit der wässrigen Brühe eines Wirkstoffes der Formel I bis zur guten Benetzung besprüht. Die Wirkstoffkonzentration betrug bis zu 500 ppm Aktivsubstanz. Die Auswertung erfolgte ca. 5 Wochen nach der Applikation des Wirkstoffs. Im Vergleich zu unbehandelten Kontrollpflanzen bewirkten die erfindungsgemässen Wirkstoffe der Formel I eine merkliche Erhöhung der Anzahl und des Gewichts der geernteten Schoten. Als besonders wirksam erwiesen sich die Verbindungen aus den Tabellen 1 und 3. Insbesondere die Verbindungen Nr. 1.1 bis 1.5, 1.15 bis 1.20, 1.25 bis 1.28, 3.20, 3.22 und 3.33 bewirkten eine Ertragssteigerung von 5 bis 12%.

Beispiel 20: Wuchshemmung bei Bodenbedecker-Pflanzen (Cover-Crops)

In Kunststoffschalen mit Erde-Torf-Sand-Gemisch (1:1:1) werden Testpflänzen der Sorte Centrosema plumieri und Centrosema pubescens aus Stecklingen angezogen. Nach dem Anwurzeln werden die Pflänzchen in 9-cm-Töpfe umgetopft und nach Bedarf gewässert. Die weitere Anzucht der Pflanzen findet im Gewächshaus bei einer Tagestemperatur von 27°C und einer Nachttemperatur von 21°C, bei einer mittleren Lichtdauer

von 14 h (6000 Lux) und einer Luftfeuchtigkeit von 70% statt. Die Testpflanzen werden auf eine Höhe von ca. 15 cm zurückgeschnitten und 7 Tage nach dem Zurückschneiden mit einer Spritzbrühe des Wirkstoffes (umgerechnet 0,3 bzw. 3 kg Aktivsubstanz je Hektar) besprüht. 4 Wochen nach Applikation wird das Wachstum der behandelten Pflanzen im Vergleich zu gestutzten, aber unbehandelten Kontrollpflanzen verglichen. Hierbei kann festgestellt werden, dass Verbindungen aus den Tabellen 1 bis 3 eine deutliche Wuchshemmung der Bodenbedecker-Pflanzen auslösen. Insbesondere die Verbindungen 1.1 bis 1.5, 1.15 bis 1.20, 1.25 bis 1.28, 3.20, 3.22 und 3.33 bewirkten eine starke Wuchshemmung und reduzierten die Zuwachsrate auf 0 bis 20%.

Beispiel 21: Seneszenzhemmung bei Getreidepflanzen

Im Gewächshaus wird Sommerweizen der Sorte "Svenno" in Töpfen mit Komposterde angesät und ohne spezielle klimatische Anforderungen gezogen. Ca. 10 Tage nach dem Auflaufen werden 10 bis 12 cm lange Primärblätter abgeschnitten und einzeln in Reagenzgläser mit 10 ml einer Wirkstoffsuspension (1,25 bis 10 ppm Aktivsubstanz) gegeben. Die Reagenzgläser werden in einem klimatisierten Raum bei 23°C, 70% relativer Luftfeuchtigkeit aufgestellt und täglich durchschnittlich 14 Stunden (10 000 Lux) bestrahlt. Die Beurteilung der Seneszenzhemmung erfolgt 7 Tage nach Einstellen der Blätter durch Vergleich des Vergilbungsgrades im Verhältnis zu noch frischen, grünen Blättern. Bei diesem Versuch kann beobachtet werden, dass Verbindungen aus den Tabellen 1 bis 3 eine deutliche Inhibierung der Seneszenz der Testpflanzen hervorrufen. Insbesondere die Verbindungen Nr. 1.1, 1.2, 1.3, 1.4, 1.5, 1.25, 1.27, 3.20 und 3.33 hemmten das Vergilben der Blätter im Versuchszeitraum um mehr als 80%.

Patentansprüche (für alle benannten Länder ausser Oesterreich)

1. Verbindungen der Formel I

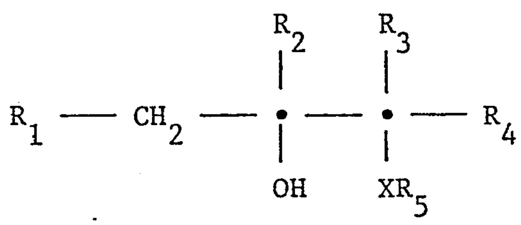

worin

$R_1$ für eine Azolylgruppe steht;

$R_2$ $C_1$-$C_4$-Alkyl, ein unsubstituiertes oder durch Halogen, Cyano, $C_1$-$C_3$-Alkyl, $C_1$-$C_3$-Alkoxy, Nitro, Rhodano, $C_1$-$C_3$-Alkylthio und/oder $C_1$-$C_3$-Haloalkyl substituiertes Aryl bedeutet;

$R_3$ und $R_4$ unabhängig voneinander für $C_1$-$C_4$-Alkyl stehen oder einer von beiden für Wasserstoff und der andere für $C_1$-$C_4$-Alkyl steht;

$R_5$ für einen unsubstituierten oder ein- oder mehrfach substituierten Rest steht, ausgewählt aus der Reihe Phenyl, Biphenyl, Benzylphenyl, Benzyloxyphenyl und Phenoxyphenyl, wobei die Substituenten aus der Reihe Halogen, Cyano, $C_1$-$C_3$-Alkyl, $C_1$-$C_5$-Alkoxy, $C_1$-$C_5$-Haloalkoxy, $C_1$-$C_4$-Alkylthio, $C_1$-$C_3$-Haloalkyl, $C_1$-$C_3$-Haloalkylthio, Nitro und/oder Rhodano ausgewählt sind; und

X Sauerstoff oder Schwefel bedeutet; unter Einschluss der Säureadditionssalze, quaternären Azoliumsalze und Metallkomplexe.

2. Verbindungen der Formel I nach Anspruch 1, worin $R_1$ für eine 1H-1,2,4-Triazolyl-, 4H-1,2,4-Triazolyl- oder eine 1H-Imidazolylgruppe steht; $R_2$ Methyl, Ethyl, iso-Propyl, tert.-Butyl, unsubstituiertes oder durch $C_1$-$C_3$-Alkyl, $C_1$-$C_3$-Alkoxy, $CF_3$, Halogen und/oder Phenyl ein- oder mehrfach substituiertes Phenyl bedeutet; $R_3$ und $R_4$ unabhängig voneinander für $C_1$-$C_2$-Alkyl stehen oder einer von beiden für Wasserstoff und der andere für $C_1$-$C_4$-Alkyl steht;
$R_5$ einen unsubstituierten oder ein- oder mehrfach durch Halogen, Cyano, $C_1$-$C_2$-Alkyl, $C_1$-$C_2$-Alkoxy, $C_1$-$C_5$-Haloalkoxy, $C_1$-$C_2$-Alkylthio,

$C_1$-$C_2$-Haloalkyl, $C_1$-$C_2$-Halothioalkyl und/oder Nitro substituierten Rest darstellt, ausgewählt aus der Reihe Phenyl, Biphenyl, Benzylphenyl und Phenoxyphenyl.

3. Verbindungen der Formel I nach einem der Ansprüche 1 oder 2, dadurch gekennzeichnet, dass $R_2$ für einen tert.-Butylrest steht.

4. Verbindungen der Formel I nach Anspruch 2, worin $R_1$ für eine 1H-1,2,4-Triazolyl-, 4H-1,2,4-Triazolyl- oder eine 1H-Imidazolylgruppe steht; $R_2$ Methyl, Ethyl, iso-Propyl, tert.-Butyl, unsubstituiertes oder durch $C_1$-$C_3$-Alkyl, $C_1$-$C_3$-Alkoxy, $CF_3$, Halogen und/oder Phenyl ein- oder mehrfach substituiertes Phenyl bedeutet; $R_3$ und $R_4$ unabhängig voneinander für $C_1$-$C_4$-Alkyl stehen; $R_5$ für einen unsubstituierten oder ein- oder mehrfach durch $C_1$-$C_2$-Alkyl, $C_1$-$C_2$-Alkoxy, Halogen, Cyano, $CF_3$, Nitro und/oder $C_1$-$C_2$-Alkylthio substituierten aromatischen Rest, ausgewählt aus der Reihe Phenyl, Biphenyl, Benzylphenyl, Benzoxyphenyl und Phenoxyphenyl steht und X Sauerstoff oder Schwefel bedeutet.

5. Verbindungen der Formel I nach Anspruch 4, worin $R_1$ für eine 1H-1,2,4-Triazolyl-, 4H-1,2,4-Triazolyl- oder eine 1H-Imidazolylgruppe steht; $R_2$ Methyl, iso-Propyl, tert.-Butyl, Phenyl, Halophenyl, Dihalophenyl oder Biphenyl bedeutet; $R_3$ Wasserstoff bedeutet und $R_4$ für $C_1$-$C_3$-Alkyl steht; $R_5$ für einen unsubstituierten oder ein- oder mehrfach durch $C_1$-$C_2$-Alkyl, $C_1$-$C_2$-Alkoxy, Halogen, Cyano, $CF_3$, Nitro und/oder $C_1$-$C_2$-Alkylthio substituierten aromatischen Rest, ausgewählt aus der Reihe Phenyl, Biphenyl, Benzylphenyl, Benzoxyphenyl und Phenoxyphenyl steht und X Sauerstoff oder Schwefel bedeutet.

6. Verbindungen der Formel I nach Anspruch 4, worin $R_1$ für eine 1H-1,2,4-Triazolyl- oder eine 4H-1,2,4-Triazolylgruppe steht; $R_2$ tert.-Butyl, Halophenyl oder Dihalophenyl bedeutet; $R_3$ Wasserstoff, Methyl oder Ethyl bedeutet, $R_4$ Methyl oder Ethyl bedeutet; $R_5$ für

ein unsubstituiertes oder ein- bis dreifach durch $C_1$-$C_2$-Alkyl, $C_1$-$C_2$-Alkoxy, Halogen, Cyano, $CF_3$, Nitro und/oder $C_1$-$C_2$-Alkylthio substituiertes Phenyl steht und X Sauerstoff oder Schwefel bedeutet.

7. Verbindungen der Formel I nach Anspruch 6, worin $R_1$ für 1H-1,2,4-Triazol oder 4H-1,2,4-Triazol steht; $R_2$ tert.-Butyl, 4-Chlorphenyl, 4-Bromphenyl, 2,4-Dichlorphenyl oder 2-Chlor-4-Bromphenyl bedeutet; $R_3$ Wasserstoff und $R_4$ Methyl oder Ethyl bedeuten; $R_5$ für ein unsubstituiertes oder ein- bis dreifach durch Methyl, Methoxy, Chlor, Brom, Fluor, Cyano, $CF_3$, Nitro oder Methylthio substituiertes Phenyl steht und X Sauerstoff bedeutet.

8. Verbindungen der Formel I nach Anspruch 7, worin $R_5$ für eine 1H-1,2,4-Triazolylgruppe steht.

9. Verbindungen der Formel I nach einem der Ansprüche 1 bis 6, dadurch gekennzeichnet, dass $R_3$ und $R_4$ Wasserstoff bedeuten.

10. Eine Verbindung, ausgewählt aus der Reihe:
1-[1H-1,2,4-Triazolyl]-2-methyl-2-hydroxy-3-[4-chlorphenoxy]-3-methyl-butan,
1-[1H-1,2,4-Triazolyl]-2-methyl-2-hydroxy-3-[2,4-dichlorphenoxy]-3-methyl-butan,
1-[1H-1,2,4-Triazolyl]-2-methyl-2-hydroxy-3-[3,4-dichlorphenoxy]-3-methyl-butan,
1-[1H-1,2,4-Triazolyl]-2-methyl-2-hydroxy-3-[4-biphenyl]-3-methyl-butan,
1-[1H-1,2,4-Triazolyl]-2-methyl-2-hydroxy-3-[4-isopropylphenoxy]-3-methyl-butan,
1-[1H-1,2,4-Triazolyl]-2-[2,4-dichlorphenyl]-2-hydroxy-3-[4-chlorphenoxy]-pentan,
1-[1H-1,2,4-Triazolyl]-2-[2,4-dichlorphenyl]-2-hydroxy-3-[4-fluorphenoxy]-pentan,

1-[1H-1,2,4-Triazolyl]-2-[2,4-dichlorphenyl]-2-hydroxy-3-[2,3-dimethylphenoxy]-pentan,

1-[1H-1,2,4-Triazolyl]-2-(2,4-dichlorphenyl]-2-hydroxy-3-[4-chlorphenoxy]-hexan,

1-[1H-1,2,4-Triazolyl]-2-[2,4-dichlorphenyl]-2-hydroxy-3-[phenoxy]-pentan,

1-[1H-1,2,4-Triazolyl]-2-[2,4-dichlorphenyl]-2-hydroxy-3-[4-bromphenoxy]-pentan,

1-[1H-1,2,4-Triazolyl]-2-[2-chlor-4-fluorphenyl]-2-hydroxy-3-[4-chlorphenoxy]-butan,

1-[1H-1,2,4-Triazolyl]-2-tert.-butyl-2-hydroxy-3-[4-chlorphenoxy]-butan,

1-[1H-1,2,4-Triazolyl]-2-tert.-butyl-2-hydroxy-3-[4-fluorphenoxy]-butan,

1-[1H-1,2,4-Triazolyl]-2-tert.-butyl-2-hydroxy-3-[4-methylphenoxy]-butan,

1-[1H-1,2,4-Triazolyl]-2-tert.-butyl-2-hydroxy-3-[4-chlorphenoxy]-pentan,

1-[4H-1,2,4-Triazolyl]-2-tert.-butyl-2-hydroxy-3-[4-chlorphenoxy]-butan,

1-[4H-1,2,4-Triazolyl]-2-tert.-butyl-2-hydroxy-3-[4-fluorphenoxy]-butan,

1-[4H-1,2,4-Triazolyl]-2-[2,4-dichlorphenyl]-2-hydroxy-3-[4-chlorphenoxy]-butan.

11. Verfahren zur Herstellung von Verbindungen der im Anspruch 1 definierten Formel I, gekennzeichnet durch Reaktion eines Oxirans der Formel II

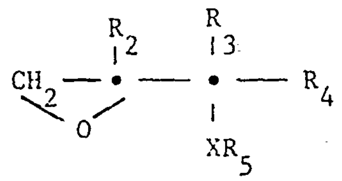

(II) ,

worin $R_2$, $R_3$, $R_4$, X und $R_5$ die unter Formel I angegebenen Bedeutungen haben mit einem Azol der Formel III

$$M - R_1 \qquad (III) \quad ,$$

worin M für Wasserstoff oder ein Metallatom steht und $R_1$ die unter Formel I in Anspruch 1 angegebenen Bedeutungen hat.

12. Verfahren nach Anspruch 11, dadurch gekennzeichnet, dass man die Reaktion in einem relativ polaren, reaktionsinerten organischen Lösungsmittel durchführt.

13. Verfahren nach Anspruch 12, dadurch gekennzeichnet, dass man als Lösungsmittel N,N-Dimethylformamid, N,N-Dimethylacetamid, Dimethylsulfoxid, Acetonitril oder Benzonitril oder ein Gemisch dieser Lösungsmittel untereinander oder zusammen mit andern üblichen reaktionsinerten organischen Lösungsmitteln einsetzt.

14. Verfahren nach Anspruch 11, dadurch gekennzeichnet, dass man die Reaktion in Gegenwart eines Kondensationsmittels oder eines säurebindenden Mittels durchführt.

15. Mittel zur Bekämpfung oder Verhütung eines Befalls durch Mikroorganismen und/oder zur Regulierung des Pflanzenwachstums, dadurch gekennzeichnet, dass es als mindestens eine aktive Komponente eine Verbindung der Formel I gemäss Anspruch 1 enthält.

16. Mittel gemäss Anspruch 15, dadurch gekennzeichnet, dass es als mindestens eine aktive Komponente eine Verbindung der Formel I gemäss Anspruch 2 enthält.

17. Mittel zur Bekämpfung von Mikroorganismen gemäss Anspruch 15, dadurch gekennzeichnet, dass es als mindestens eine aktive Komponente eine Verbindung der Formel I gemäss Anspruch 3 enthält.

18. Mittel gemäss Anspruch 15, dadurch gekennzeichnet, dass es als mindestens eine aktive Komponente eine Verbindung der Formel I gemäss einem der Ansprüche 4 bis 10 enthält.

19. Mittel nach einem der Ansprüche 15 bis 18, dadurch gekennzeichnet, dass es 0,1 bis 99% eines Wirkstoffs der Formel I, 99,9 bis 1% eines festen oder flüssigen Zusatzstoffes und 0 bis 25% eines Tensids enthält.

20. Mittel nach Anspruch 19, dadurch gekennzeichnet, dass es 0,1 bis 95% eines Wirkstoffes der Formel I, 99,8 bis 5% eines festen oder flüssigen Zusatzstoffes und 0,1 bis 25% eines Tensides enthält.

21. Verfahren zur Herstellung eines wie in den Ansprüchen 15 bis 20 beanspruchten agrochemischen Mittels, dadurch gekennzeichnet, dass man mindestens eine gemäss einem der Ansprüche 1 bis 10 definierte Verbindung der Formel I mit geeigneten festen oder flüssigen Zusatzstoffen und Tensiden innig vermischt.

22. Verfahren zur Bekämpfung oder Verhütung eines Befalls von Kulturpflanzen durch phytopathogene Mikroorganismen und/oder zur Regulierung des Pflanzenwachstums, dadurch gekennzeichnet, dass man eine gemäss den Ansprüchen 1 bis 10 definierte Verbindung der Formel I auf die Pflanze oder deren Standort appliziert.

23. Verwendung von Verbindungen der Formel I gemäss Anspruch 1 zur Bekämpfung und/oder zur präventiven Verhütung eines Befalls von Mikroorganismen und/oder zur Regulierung des Pflanzenwachstums.

24. Verwendung nach Anspruch 23 von Verbindungen der Formel I gemäss einem der Ansprüche 2 bis 10.

25. Verwendung gemäss einem der Ansprüche 23 bis 24, dadurch gekennzeichnet, dass es sich bei den Mikroorganismen um phytopathogene Pilze handelt.

26. Verwendung gemäss Anspruch 25 gegen Pilze aus den Klassen Ascomycetes, Basidiomycetes oder Fungi imperfecti.

27. Verwendung gemäss Anspruch 23 oder 24 zur Wuchshemmung im Sinne einer Erhöhung der Knickfestigkeit und Halmverkürzung bei Getreidesorten.

28. Verwendung gemäss Anspruch 27, dadurch gekennzeichnet, dass es sich bei den Getreidesorten um Hafer, Weizen, Gerste oder Roggen handelt.

29.Verwendung gemäss Anspruch 23 oder 24 zur Wuchshemmung bei Gräsern.

30. Verwendung gemäss Anspruch 23 oder 24 zur Wuchshemmung bei Bodenbedeckerpflanzen.

31. Verwendung gemäss Anspruch 23 oder 24 zur Wuchsregulierung bei Leguminosen im Sinne einer Ertragssteigerung.

32. Verwendung gemäss Anspruch 31, dadurch gekennzeichnet, dass es sich um Soja handelt.

33. Die Oxirane der Formel II

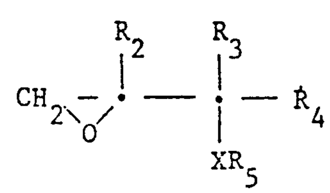

(II),

worin $R_2$, $R_3$, $R_4$, X und $R_5$ die in Anspruch 1 unter Formel I angegebenen Bedeutungen haben.

Patentansprüche (für Oesterreich)

1. Mittel zur Bekämpfung oder Verhütung eines Befalls durch Mikroorganismen und/oder zur Regulierung des Pflanzenwachstums, dadurch gekennzeichnet, dass es neben üblichen Trägermaterialien und applikationsfördernden Zusätzen als mindestens eine aktive Komponente eine Verbindung der Formel I enthält,

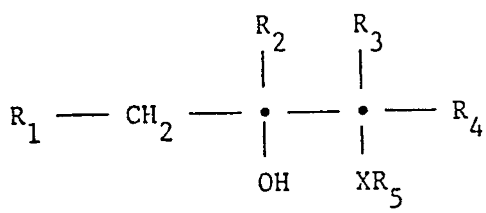

(I) ,

worin

$R_1$ für eine Azolylgruppe steht;

$R_2$ $C_1$-$C_4$-Alkyl, ein unsubstituiertes oder durch Halogen, Cyano, $C_1$-$C_3$-Alkyl, $C_1$-$C_3$-Alkoxy, Nitro, Rhodano, $C_1$-$C_3$-Alkylthio und/oder $C_1$-$C_3$-Haloalkyl substituiertes Aryl bedeutet;

$R_3$ und $R_4$ unabhängig voneinander für $C_1$-$C_4$-Alkyl stehen oder einer von beiden für Wasserstoff und der andere für $C_1$-$C_4$-Alkyl steht;

$R_5$ für einen unsubstituierten oder ein- oder mehrfach substituierten Rest steht, ausgewählt aus der Reihe Phenyl, Biphenyl, Benzylphenyl, Benzyloxyphenyl und Phenoxyphenyl, wobei die Substituenten aus der Reihe Halogen, Cyano, $C_1$-$C_3$-Alkyl, $C_1$-$C_5$-Alkoxy, $C_1$-$C_5$-Haloalkoxy, $C_1$-$C_4$-Alkylthio, $C_1$-$C_3$-Haloalkyl, $C_1$-$C_3$-Haloalkylthio, Nitro und/oder Rhodano ausgewählt sind; und

X Sauerstoff oder Schwefel bedeutet; unter Einschluss der Säureadditionssalze, quaternären Azoliumsalze und Metallkomplexe.

2. Mittel nach Anspruch 1, dadurch gekennzeichnet, dass es als mindestens eine aktive Komponente eine Verbindung der Formel I enthält, worin $R_1$ für eine 1H-1,2,4-Triazolyl-, 4H-1,2,4-Triazolyl- oder eine 1H-Imidazolylgruppe steht; $R_2$ Methyl, Ethyl, iso-Propyl, tert.-Butyl, unsubstituiertes oder durch $C_1$-$C_3$-Alkyl, $C_1$-$C_3$-Alkoxy, $CF_3$, Halogen und/oder Phenyl ein- oder mehrfach substituiertes Phenyl bedeutet; $R_3$ und $R_4$ unabhängig voneinander für $C_1$-$C_2$-Alkyl stehen oder einer von beiden für Wasserstoff und der andere für $C_1$-$C_4$-Alkyl steht;
$R_5$ einen unsubstituierten oder ein- oder mehrfach durch Halogen, Cyano, $C_1$-$C_2$-Alkyl, $C_1$-$C_2$-Alkoxy, $C_1$-$C_5$-Haloalkoxy, $C_1$-$C_2$-Alkylthio, $C_1$-$C_2$-Haloalkyl, $C_1$-$C_2$-Halothioalkyl und/oder Nitro substituierten Rest darstellt, ausgewählt aus der Reihe Phenyl, Biphenyl, Benzylphenyl und Phenoxyphenyl.

3. Mittel nach einem der Ansprüche 1 oder 2, dadurch gekennzeichnet, dass es als mindestens eine aktive Komponente eine Verbindung der Formel I enthält, worin $R_2$ für den tert.-Butylrest steht.

4. Mittel nach Anspruch 2, dadurch gekennzeichnet, dass es als mindestens eine aktive Komponente eine Verbindung der Formel I enthält, worin $R_1$ für eine 1H-1,2,4-Triazolyl-, 4H-1,2,4-Triazolyl- oder eine 1H-Imidazolyl gruppe steht; $R_2$ Methyl, Ethyl, iso-Propyl, tert.-Butyl, unsubstituiertes oder durch $C_1$-$C_3$-Alkyl, $C_1$-$C_3$-Alkoxy, $CF_3$, Halogen und/oder Phenyl ein- oder mehrfach substituiertes Phenyl bedeutet; $R_3$ und $R_4$ unabhängig voneinander für $C_1$-$C_4$-Alkyl stehen; $R_5$ für einen unsubstituierten oder ein- oder mehrfach durch $C_1$-$C_2$-Alkyl, $C_1$-$C_2$-Alkoxy, Halogen, Cyano, $CF_3$, Nitro und/oder $C_1$-$C_2$-Alkylthio substituierten aromatischen Rest, ausgewählt aus der Reihe Phenyl, Biphenyl, Benzylphenyl, Benzoxyphenyl und Phenoxyphenyl steht und X Sauerstoff oder Schwefel bedeutet.

5. Mittel nach Anspruch 4, dadurch gekennzeichnet, dass es als mindestens eine aktive Komponente eine Verbindung der Formel I enthält, worin $R_1$ für eine 1H-1,2,4-Triazolyl-, 4H-1,2,4-Triazolyl- oder eine 1H-Imidazolylgruppe steht; $R_2$ Methyl, iso-Propyl, tert.-Butyl, Phenyl, Halophenyl, Dihalophenyl oder Biphenyl bedeutet; $R_3$ Wasserstoff bedeutet und $R_4$ für $C_1$-$C_3$-Alkyl steht; $R_5$ für einen unsubstituierten oder ein- oder mehrfach

durch $C_1$-$C_2$-Alkyl, $C_1$-$C_2$-Alkoxy, Halogen, Cyano, $CF_3$, Nitro und/oder $C_1$-$C_2$-Alkylthio substituierten aromatischen Rest, ausgewählt aus der Reihe Phenyl, Biphenyl, Benzylphenyl, Benzoxyphenyl und Phenoxyphenyl steht und X Sauerstoff oder Schwefel bedeutet.

6. Mittel nach Anspruch 4, dadurch gekennzeichnet, dass es als mindestens eine aktive Komponente eine Verbindung der Formel I enthält, worin $R_1$ für eine 1H-1,2,4-Triazolyl- oder eine 4H-1,2,4-Triazolylgruppe steht; $R_2$ tert.-Butyl, Halophenyl oder Dihalophenyl bedeutet; $R_3$ Wasserstoff, Methyl oder Ethyl bedeutet, $R_4$ Methyl oder Ethyl bedeutet; $R_5$ für ein unsubstituiertes oder ein- bis dreifach durch $C_1$-$C_2$-Alkyl, $C_1$-$C_2$-Alkoxy, Halogen, Cyano, $CF_3$, Nitro und/oder $C_1$-$C_2$-Alkylthio substituiertes Phenyl steht und X Sauerstoff oder Schwefel bedeutet.

7. Mittel nach Anspruch 6, dadurch gekennzeichnet, dass es als mindestens eine aktive Komponente eine Verbindung der Formel I enthält, worin $R_1$ für 1H-1,2,4-Triazol oder 4H-1,2,4-Triazol steht; $R_2$ tert.-Butyl, 4-Chlorphenyl, 4-Bromphenyl, 2,4-Dichlorphenyl oder 2-Chlor-4-Bromphenyl bedeutet; $R_3$ Wasserstoff und $R_4$ Methyl oder Ethyl bedeuten; $R_5$ für ein unsubstituiertes oder ein- bis dreifach durch Methyl, Methoxy, Chlor, Brom, Fluor, Cyano, $CF_3$, Nitro oder Methylthio substituiertes Phenyl steht und X Sauerstoff bedeutet.

8. Mittel nach Anspruch 7, dadurch gekennzeichnet, dass es als mindestens aktive Komponente eine Verbindung der Formel I enthält, worin $R_5$ für eine 1H-1,2,4-Triazolylgruppe steht.

9. Mittel nach einem der Ansprüche 1 bis 6, dadurch gekennzeichnet, dass es als mindestens eine aktive Komponente eine Verbindung der Formel I enthält, worin $R_3$ und $R_4$ für Wasserstoff stehen.

10. Mittel nach einem der Ansprüche 1 bis 3, dadurch gekennzeichnet, dass es als mindestens eine aktive Komponente eine der nachfolgend aufgeführten Verbindungen der Formel I enthält, nämlich

1-[1H-1,2,4-Triazolyl]-2-methyl-2-hydroxy-3-[4-chlorphenoxy]-3-methyl-butan,

1-[1H-1,2,4-Triazolyl]-2-methyl-2-hydroxy-3-[2,4-dichlorphenoxy]-3-methyl-butan,

1-[1H-1,2,4-Triazolyl]-2-methyl-2-hydroxy-3-[3,4-dichlorphenoxy]-3-methyl-butan,

1-[1H-1,2,4-Triazolyl]-2-methyl-2-hydroxy-3-[4-biphenyl]-3-methyl-butan,

1-[1H-1,2,4-Triazolyl]-2-methyl-2-hydroxy-3-[4-isopropylphenoxy]-3-methyl-butan,

1-[1H-1,2,4-Triazolyl]-2-[2,4-dichlorphenyl]-2-hydroxy-3-[4-chlorphenoxy]-pentan,

1-[1H-1,2,4-Triazolyl]-2-[2,4-dichlorphenyl]-2-hydroxy-3-[4-fluorphenoxy]-pentan,

1-[1H-1,2,4-Triazolyl]-2-[2,4-dichlorphenyl]-2-hydroxy-3-[2,3-dimethylphenoxy]-pentan,

1-[1H-1,2,4-Triazolyl]-2-(2,4-dichlorphenyl]-2-hydroxy-3-[4-chlorphenoxy]-hexan,

1-[1H-1,2,4-Triazolyl]-2-[2,4-dichlorphenyl]-2-hydroxy-3-[phenoxy]-pentan,

1-[1H-1,2,4-Triazolyl]-2-[2,4-dichlorphenyl]-2-hydroxy-3-[4-bromphenoxy]-pentan,

1-[1H-1,2,4-Triazolyl]-2-[2-chlor-4-fluorphenyl]-2-hydroxy-3-[4-chlorphenoxy]-butan,

1-[1H-1,2,4-Triazolyl]-2-tert.-butyl-2-hydroxy-3-[4-chlorphenoxy]-butan,

1-[1H-1,2,4-Triazolyl]-2-tert.-butyl-2-hydroxy-3-[4-fluorphenoxy]-butan,

1-[1H-1,2,4-Triazolyl]-2-tert.-butyl-2-hydroxy-3-[4-methylphenoxy]-butan,

1-[1H-1,2,4-Triazolyl]-2-tert.-butyl-2-hydroxy-3-[4-chlorphenoxy]-pentan,

1-[4H-1,2,4-Triazolyl]-2-tert.-butyl-2-hydroxy-3-[4-chlorphenoxy]-butan,

1-[4H-1,2,4-Triazolyl]-2-tert.-butyl-2-hydroxy-3-[4-fluorphenoxy]-butan,
1-[4H-1,2,4-Triazolyl]-2-[2,4-dichlorphenyl]-2-hydroxy-3-[4-chlor-phenoxy]-butan.

11. Verfahren zur Herstellung von Verbindungen der im Anspruch 1 definierten Formel I, gekennzeichnet durch Reaktion eines Oxirans der Formel II

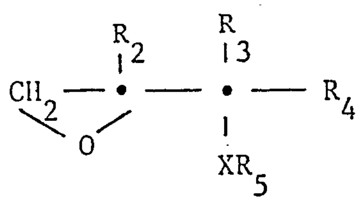

(II) ,

worin $R_2$, $R_3$, $R_4$, X und $R_5$ die unter Formel I angegebenen Bedeutungen haben mit einem Azol der Formel III

$$M — R_1 \qquad (III) ,$$

worin M für Wasserstoff oder ein Metallatom steht und $R_1$ die unter Formel I in Anspruch 1 angegebenen Bedeutungen hat.

12. Verfahren nach Anspruch 11, dadurch gekennzeichnet, dass man die Reaktion in einem relativ polaren, reaktionsinerten organischen Lösungsmittel durchführt.

13. Verfahren nach Anspruch 12, dadurch gekennzeichnet, dass man als Lösungsmittel N,N-Dimethylformamid, N,N-Dimethylacetamid, Dimethylsulfoxid, Acetonitril oder Benzonitril oder ein Gemisch dieser Lösungsmittel untereinander oder zusammen mit andern üblichen reaktionsinerten organischen Lösungsmitteln einsetzt.

14. Verfahren nach Anspruch 11, dadurch gekennzeichnet, dass man die Reaktion in Gegenwart eines Kondensationsmittels oder eines säurebindenden Mittels durchführt.

15. Verfahren zur Herstellung eines wie in den Ansprüchen 1 bis 10 beanspruchten agrochemischen Mittels, dadurch gekennzeichnet, dass man mindestens eine gemäss einem der Ansprüche 1 bis 10 definierte Verbindung der Formel I mit geeigneten festen oder flüssigen Zusatzstoffen und Tensiden innig vermischt.

16. Verfahren zur Bekämpfung oder Verhütung eines Befalls von Kulturpflanzen durch phytopathogene Mikroorganismen und/oder zur Regulierung des Pflanzenwachstums, dadurch gekennzeichnet, dass man eine gemäss den Ansprüchen 1 bis 10 definierte Verbindung der Formel I auf die Pflanze oder deren Standort appliziert.

17. Verwendung von Verbindungen der Formel I gemäss Anspruch 1 zur Bekämpfung und/oder zur präventiven Verhütung eines Befalls von Mikroorganismen und/oder zur Regulierung des Pflanzenwachstums.

18. Verwendung nach Anspruch 17 von Verbindungen der Formel I gemäss einem der Ansprüche 2 bis 10.

19. Verwendung gemäss einem der Ansprüche 17 bis 18, dadurch gekennzeichnet, dass es sich bei den Mikroorganismen um phytopathogene Pilze handelt.

20. Verwendung gemäss Anspruch 19 gegen Pilze aus den Klassen Ascomycetes, Basidiomycetes oder Fungi imperfecti.

21. Verwendung gemäss Anspruch 17 oder 18 zur Wuchshemmung im Sinne einer Erhöhung der Knickfestigkeit und Halmverkürzung bei Getreidesorten.

22. Verwendung gemäss Anspruch 21, dadurch gekennzeichnet, dass es sich bei den Getreidesorten um Hafer, Weizen, Gerste oder Roggen handelt.

23. Verwendung gemäss Anspruch 17 oder 18 zur Wuchshemmung bei Gräsern.

24. Verwendung gemäss Anspruch 17 oder 18 zur Wuchshemmung bei Bodenbedeckerpflanzen.

25. Verwendung gemäss Anspruch 17 oder 18 zur Wuchsregulierung bei Leguminosen im Sinne einer Ertragssteigerung.

26. Verwendung gemäss Anspruch 25, dadurch gekennzeichnet, dass es sich um Soja handelt.

27. Verwendung der Oxirane der Formel II

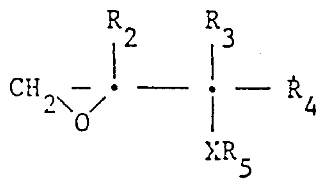

(II),

worin $R_2$, $R_3$, $R_4$, X und $R_5$ die in Anspruch 1 unter Formel I angegebenen Bedeutungen haben, zur Herstellung einer der in Anspruch 1 definierten Wirkstoffe der Formel I.